# EUROPEAN PATENT SPECIFICATION

(11) **EP 0 742 721 B1**
(45) Date of publication and mention of the grant of the patent: **06.05.1999**
(21) Application number: 94926660.5
(22) Date of filing: 02.09.1994
(51) Int. Cl.: A61K 39/395, A61K 38/17, G01N 33/68, C12P 21/08

(54) **METHODS OF PROLONGED SUPPRESSION OF HUMORAL IMMUNITY**
VERFAHREN ZUR VERLAENGERTER UNTERDRUECKUNG DER HUMORALEN IMMUNITAET
PROCEDES DE SUPPRESSION PROLONGEE DE L'IMMUNITE HUMORALE

(30) Priority: 02.09.1993 US 115990; 25.04.1994 US 232929
(43) Date of publication of application: 20.11.1996
(73) Proprietor: Trustees of Dartmouth College, Hanover, NH 03755 (US)
(72) Inventor: NOELLE, Randolph, J., Cornish, NH 03745 (US)
(74) Representative: Daniels, Jeffrey Nicholas
(86) International application number: US9409872
(87) International publication number: WO9506480

(56) References cited:
- EP-A- 0 555 880
- WO-A-93/08207
- JOURNAL OF CLINICAL IMMUNOLOGY, vol.13, no.3, May 1993, NEW YORK NY, USA pages 165 - 174 L. MARSHALL ET AL. 'The molecular basis for T cell help in humoral immunity: CD40 and its ligand, gp39.'
- IMMUNOLOGY TODAY, vol.13, no.11, November 1992, AMSTERDAM, THE NETHERLANDS pages 431 - 433 R. NOELLE ET AL. 'CD40 and its ligand, an essential ligand-receptor pair for thymus-dependent B-cell activation.'
- PROCEEDINGS OF THE NATIONAL ACADEMY OF SCIENCES OF THE USA, vol.89, no.14, 15 July 1992, WASHINGTON DC, USA pages 6550 - 6554 R. NOELLE ET AL. 'A 39-kDa protein on activated helper T cells binds CD40 and transduces the signal for cognate activation of B cells.' cited in the application
- THE JOURNAL OF IMMUNOLOGY, vol.149, no.2, 15 July 1992, BALTIMORE MD, USA pages 655 - 660 W. FANSLOW ET AL. 'Soluble forms of CD40 inhibit biologic responses of human B cells.'
- THE JOURNAL OF EXPERIMENTAL MEDICINE, vol.178, no.5, 1 November 1993, NEW YORK NY, USA pages 1567 - 1575 T. FOY ET AL. 'In vivo CD40-gp39 interactions are essential for thymus-dependent humoral immunity. II. Prolonged suppression of the humoral immune response by an antibody to the ligand for CD40, gp39.'
- THE JOURNAL OF EXPERIMENTAL MEDICINE, vol.180, no.1, 1 July 1994, NEW YORK NY, USA pages 157 - 163 T. FOY ET AL. 'gp39-CD40 interactions are essential for germinal center formation and the development of B cell memory.'

## Description

### Background of the Invention

The immune system is capable of producing two types of antigen-specific responses to foreign antigen. Cell-mediated immunity is the term used to refer to effector functions of the immune system mediated by T lymphocytes. Humoral immunity is the term used to refer to production of antigen-specific antibodies by B lymphocytes. It has long been appreciated that the development of humoral immunity against most antigens requires not only antibody-producing B lymphocytes but also the involvement of helper T (hereafter Th) lymphocytes. Mitchison, *Eur. J Immunol.*, 1:18-25 (1971); Claman and Chaperon, *Transplant Rev*., 1 92-119 (1969); Katz et al., *Proc. Natl. Acad. Sci. USA*. 70:2624-2629 (1973); Raff et al.. *Nature,* 226:1257-1260 (1970). Certain signals, or "help", are provided by Th cells in response to stimulation by thymus-dependent (hereafter TD) antigens. While some B lymphocyte help is mediated by soluble molecules released by Th cells (for instance lymphokines such as IL-4 and IL-5), activation of B cells also requires a contact-dependent interaction between B cells and Th cells. Hirohata et al., *J Immunol*., 140:3736-3744 (1988); Bartlett et al.. *J. Immunol*., 143:1745-1754 (1989). This indicates that B cell activation involves an obligatory interaction between cell surface molecules on B cells and Th cells. Such an interaction is further supported by the observation that isolated plasma membranes of activated T cells can provide helper functions necessary for B cell activation. Brian. *Proc. Natl. Acad. Sci. USA*, 85:564-568 (1988); Hodgkin et al., *J. Immunol*., 145:2025-2034 (1990): Noelle et al.. *J Immunol*.. 146:1118-1124 (1991).

A cell surface molecule, CD40, has been identified on immature and mature B lymphocytes which, when crosslinked by antibodies, induces B cell proliferation. Valle et al.. *Eur. J Immunol*., 19:1463-1467 (1989); Gordon et al.. *J Immunol*.. 140:1425-1430 (1988); Gruber et al., *J Immunol*., 142:4144-4152 (1989). CD40 has been molecularly cloned and characterized. Stamenkovic et al.. *EMBO J*: 8:1403-1410 (1989). A ligand for CD40, gp39 (also called CD40 ligand or CD40L) has also been molecularly cloned and characterized. Armitage et al., *Nature*, 357:80-82 (1992); Lederman et al.. *J. Exp. Med*.. 175:1091-1101 (1992); Hollenbaugh et al., *EMBO J*, 11:4313-4319 (1992). The gp39 protein is expressed on activated, but not resting. CD4⁺ Th cells. Spriggs et al.. *J Exp. Med*.. 176:1543-1550 (1992); Lane et al.. *Eur. J Immunol*.. 22:2573-2578 (1992): Roy et al., *J Immunol*., 151:1-14 (1993). Cells transfected with the gp39 gene and expressing the gp39 protein on their surface can trigger B cell proliferation and, together with other stimulatory signals, can induce antibody production. Armitage et al.. *Nature*. 357:80-82 (1992); Hollenbaugh et al., *EMBO J*., 11:4313-4319 (1992).

EP-A-0555880 relates to a counter-receptor, termed CD40 CR, for the CD40 B-cell antigen and to soluble ligands for this receptor, including fusion molecules comprising at least a portion CD40 protein.

WO93/08207 relates to the cloning of a murine and a human cytokine that binds to a human CD40 having both agonist and antagonist activity in soluble and membrane-bound forms.

Marshall *et al* discuss in J Clin Immunol 13(3):165-174 of 1993 the molecular basis for T-cell help in humoral immunity and interactions between CD40 and its ligand, gp39.

Noelle *et al* discuss in Immunology Today 13(11):431-433 of 1992 CD40 and its ligand as an essential ligand-receptor pair for thymus-dependent B-cell activation.

While the induction of a humoral immune response is an important host defense mechanism, in certain situations it would be beneficial to suppress antibody production against a particular antigen. For example, suppression of a humoral response against an allergen could prevent or reduce an allergic response in an individual. Additionally, when a therapeutic antibody is administered, suppressing a humoral response against the antibody could prolong the therapeutic efficacy of the antibody.

### Summary of the Invention

One approach to suppressing humoral immunity is to inhibit B cell activation. The current invention pertains to methods for inhibiting a humoral immune response to a TD antigen *in vivo* by inhibiting the ability of a Th cell to stimulate a B cell, thereby interfering with B cell activation and antibody production. The invention is based, at least in part, on necessity for an *in vivo* interaction between gp39 on a Th cell and CD40 on a B cell for subsequent activation of the B cell.

Accordingly, the present invention provides a pharmaceutical composition comprising a thymus-dependent (TD) antigen and a gp39 antagonist.

The present invention further provides use of a gp39 antagonist for the preparation of a medicament for inhibiting a humoral immune response to a thymus-dependent (TD) antigen *in vivo*, wherein the TD antigen is administered to a subject with the antagonist.

The present invention further provides use of a gp39 antagonist for the preparation of a medicament for providing for suppression of an antigen specific IgE response to an administered thymus -dependent (TD) antigen *in vivo*, wherein the gp39 antagonist is administered to a subject exposed to the TD antigen, and suppression of antibody production against the administered antigen is maintained after administration of the gp39 antagonist has terminated.

The present invention further provides use of a gp39 antagonist for the preparation of a medicament for providing for suppression of an antigen specific IgE response to an administered thymus-dependent (TD) antigen *in vivo*, wherein the gp39 antagonist is administered to a subject exposed to the TD antigen, and suppression of antibody production against the administered antigen is maintained after administration of the gp39 antagonist has terminated.

The present invention further provides use of a gp39 antagonist for the preparation of a medicament for providing for suppression of a humoral response to an administered allergen to which a subject is exposed, wherein suppression of antibody production against the administered antigen is maintained after administration of the gp39 antagonist has terminated.

The present invention further provides use of a gp39 antagonist for the preparation of a medicament for immunosuppressing function of activated helper T cells induced by a thymus-dependent (TD) antigen *in vivo*, wherein the TD antigen is administered to a subject with the gp39 antagonist.

The present invention further provides use of a gp39 antagonist for the preparation for a composition for determining whether an antigen is a thymus-dependent (TD) antigen or a thymus-independent type II (TI-2) antigen, wherein
(a) the antigen to be tested is administered to a subject with the gp39 antagonist;
(b) humoral immune responses are measured against the antigen; and
(c) the antigen is determined to be a TD antigen by an absence of humoral immune responses or the antigen is determined to be a TI-2 antigen by a presence of humoral immune responses.

The present invention further provides use of a gp39 antagonist for the preparation of a medicament for providing for the suppression of a humoral response to an administered thymus-dependent (TD) antigen which TD antigen comprises a therapeutic agent to which a subject is exposed.

The present invention further provides use of a gp39 antagonist for the preparation of a medicament for providing for the suppression of an administered antigen specific IgE response to a thymus-dependent (TD) antigen, wherein such TD antigen comprises a therapeutic agent to which a subject is exposed.

The gp39 antagonist which is administered can be an antibody directed against gp39. In a preferred embodiment, the gp39 antagonist is a monoclonal antibody, such as a anti-human gp39 antibody or an anti-mouse gp39 antibody (e.g., MR1). Chimeric antibodies, humanized antibodies and antibody fragments are also within the scope of the invention. Alternatively, the gp39 antagonist can be a soluble form of the gp39 ligand CD40. Soluble fusion proteins of CD40 are also encompassed by the invention.

The humoral immune response inhibited by the methods of the current invention can be a primary humoral immune response, in the case of initial exposure to an antigen, or a secondary humoral immune response, in the case of reexposure to a previously encountered antigen. For example, the methods described herein can be used to inhibit production of antigen-specific IgM antibodies. IgG antibodies, IgD antibodies and/or IgE antibodies. In addition, the methods provide for prolonged suppression of humoral immune responses *in vivo*.

One aspect of the invention provides methods for inhibiting humoral immune responses to TD antigens. Antigens embraced by the invention include antigens for which specific antibody production requires interaction of gp39 with a ligand on the surface of B cells (e.g., CD40). TD antigens generally include proteinaceous antigens. In preferred embodiments of the invention, the antigen is a therapeutic antibody, drug, allergen or foreign cell. The methods of the present invention also are effective for inhibiting humoral immune responses to a TD antigen while preserving humoral immune responses to thymus-independent type II (hereafter TI-2) antigens.

Another aspect of the invention pertains to methods for specifically inhibiting the helper function of activated Th cells *in vivo* by interfering with the interaction of gp39 with a ligand on the surface of B cells (e.g., CD40) by administering a gp39 antagonist. According to this method, helper function of activated Th cells is inhibited *in vivo* without deleting or anergizing Th cells.

The invention further pertains to methods of inhibiting humoral immune responses *in vivo* by a combined administration of a gp39 antagonist and another immunosuppressive agent. Other immunosuppressive agents which can be provided in conjunction with a gp39 antagonist include cytokine inhibitors, inhibitors of the CD28/CTLA4 T cell costimulatory pathway, or immunosuppressive drugs.

A still further aspect of the present invention is a method for determining whether an antigen is a TD or TI-2 antigen. This can be determined by whether or not humoral immune responses to the antigen *in vivo* can be inhibited by administration of a gp39 antagonist.

### Brief Description of the Drawings

Figure 1A is a bar graph depicting suppression of primary anti-SRBC IgM antibody production by *in vivo* anti-gp39 treatment.
Figure 1B is a graph depicting prolonged suppression of primary anti-SRBC IgM antibody production after short-term *in vivo* anti-gp39 treatment.
Figure 2A is a bar graph depicting suppression of secondary anti-KLH antibody production (various isotypes) by in vivo anti-gp39 treatment. Antibody titers were measured 7 days after antigen challenge.
Figure 2B is a bar graph depicting suppression of secondary anti-KLH antibody production (various isotypes) by *in vivo* anti-gp39 treatment. Antibody titers were measured 14 days after antigen challenge.
Figure 3 is two bar graphs depicting suppression of primary anti-ChiL6 IgM antibody production (left) and secondary anti-ChiL6 IgG1 antibody production (right) by i*n vivo* anti-gp39 treatment.
Figure 4A is a bar graph depicting suppression of primary anti-TNP IgM antibody production by immunization with TNP-SRBC and *in vivo* anti-gp39 treatment.
Figure 4B is a bar graph depicting lack of suppression of primary anti-TNP IgM antibody production by immunization with TNP-Ficoll and *in vivo* anti-gp39 treatment.
Figure 5 is a bar graph depicting intact helper activity of T cells previously exposed to anti-gp39 treatment *in vivo* upon adoptive transfer to untreated mice, demonstrating that anti-gp39 administration does not functionally delete Th cells.
Figure 6A is a Western blot depicting anti-gp39 antibody present in serum 7,14 and 21 days after *in vivo* administration.
Figure 6B is a graph depicting percent of remaining anti-gp39 activity in serum 7,14 and 21 days after *in vivo* administration.
Figures 7A, B and C are flow cytometic profiles depicting the staining of 6 hour activated human peripheral blood lymphocytes with either CD40Ig (panel A), mAb 4D9-8 (panel B) or mAb 4D9-9 (panel C).
Figures 8A, B and C are flow cytometic profiles depicting the staining of 6 hour activated human peripheral blood lymphocytes cultured in the presence of cycloporin A stained with either mAb 4D9-8 (panel A), mAb 4D9-9 (panel B) or CD40Ig (panel C).
Figures 9A and B are flow cytometric profiles depicting the staining of 6 hour activated human peripheral blood lymphocytes with CD40Ig in the presence of unlabeled mAb 4D9-8 (panel A) or unlabeled mAb 4D9-9 (Panel B).
Figure 10 is a graphic representation of the inhibition of human B cell proliferation induced by soluble gp39 and IL-4 when cells are cultured in the presence of anti-human gp39 mAbs 4D9-8, 4D9-9, 24-31, 24-43, 89-76 or 89-79.
Figure 11 is a graphic representation of the inhibition of an allo-specific mixed lymphocyte response when cells are cultured in the presence of anti-human gp39 mAbs 24-31 or 89-79.

### Detailed Description of the Invention

The generation of humoral immunity to thymus-dependent (TD) antigens requires not only B lymphocytes, which can produce specific antibodies against an antigen, but also contributions from Th cells which are necessary for activation of B lymphocytes. Although T helper cell function does involve production of cytokines utilized by B lymphocytes, the Th cell requirement for B cell activation cannot be eliminated by providing exogenous cytokines to B cells. Rather, a contact-dependent, cell membrane- mediated interaction between B cells and Th cells is integral to induction of humoral responses. A receptor-ligand pair involved in this interaction, CD40 and gp39, has been identified. CD40 is present on B cells and has the ability to bind to gp39, which is induced on Th cells upon activation, leading to stimulation of the B cells and ultimately production of specific antibodies. Disruption of the CD40-gp39 interaction offers a means of interfering with the generation of a specific humoral immune response.

Accordingly, this invention pertains to methods of inhibiting humoral immune responses against a TD antigen *in vivo*. Humoral immune responses are inhibited by interfering with the interaction of a molecule on a Th cell which mediates contact-dependent helper effector function and it's ligand on the surface of a B lymphocyte. In a preferred embodiment, humoral immune responses are inhibited by interfering with the interaction of gp39 on a T cell and CD40 on a B cell exposed to the TD antigen through administration of a gp39 antagonist to a subject *in vivo*. In one embodiment, the B cell is exposed to the TD antigen by administration of the antigen *in vivo* with the gp39 antagonist. Preferably, this TD antigen is a therapeutic agent, for example a therapeutic antibody or drug, which is administered to the patient for therapeutic treatment and for which inhibiting humoral immune responses against it can result in prolonged therapeutic efficacy of the agent. In another embodiment, the TD antigen is an antigen to which a subject is exposed to enviromentally, for example an allergan, in which a humoral immune response is detrimental to the subject, for example results in an allergic reaction. Inhibition of a humoral immune response in this situation would be therapeutically beneficial to the subject.

### I. GP39 ANTAGONISTS

According to the methods of the invention, a gp39 antagonist is administered to a subject to interfere with the interaction of gp39 on 7 cells with a gp39 ligand on B cells. A gp39 antagonist is defined as a molecule which interferes with this interaction. The gp39 antagonist can be an antibody directed against gp39 (e.g., a monoclonal antibody against gp39), fragments or derivative of an antibody directed against gp39 (e.g., Fab or F(ab)'2 fragments, chimeric antibodies or humanized antibodies), soluble forms of a gp39 ligand (e.g., soluble CD40), soluble forms of a fusion protein of a gp39 ligand (e.g., soluble CD40Ig), or pharmaceutical agents which disrupt the gp39-CD40 interaction.

A mammal, (e.g., a mouse, hamster, or rabbit) can be immunized with an immunogenic form of gp39 protein or protein fragment (e.g., peptide fragment) which elicits an antibody response in the mammal. A cell which expresses gp39 on its surface can also be used as the immunogen. Alternative immunogens include purified gp39 protein or protein fragments. gp39 can be purified from a gp39-expressing cell by standard purification techniques; gp39 cDNA (Armitage et al., *Nature*, 357:80-82 (1992); Lederman et al., *J Exp. Med.*,175:1091-1101(1992); Hollenbaugh et al., *EMBO J,* 11:4313-4319 (1992) can be expressed in a host cell, e.g. bacteria or a mammalian cell line, and gp39 protein purified. gp39 peptides can be synthesized based upon the amino acid sequence of gp39 (Armitage et al., *Nature*, 357:80-82(1992); Lederman et al., *J Exp. Med*., 175:1091-1101(1992); Hollenbaugh et al., *EMBO J*., 11:4313-4319 (1992)). Techniques for conferring immunogenicity on a protein include conjugation to carriers or other techniques well known in the art. For example, the protein can be administered in the presence of adjuvant. The progress of immunization can be monitored by detection of antibody titers in plasma or serum. Standard ELISA or other immunoassay can be used with the immunogen as antigen to assess the levels of antibodies.

Following immunization, antisera can be obtained and, if desired, polyclonal antibodies isolated from the sera. To produce monoclonal antibodies, antibody producing cells (lymphocytes) can be harvested from an immunized animal and fused with myeloma cells by standard somatic cell fusion procedures thus immortalizing these cells and yielding hybridoma cells. Such techniques are well known in the art. For example, the hybridoma technique originally developed by Kohler and Milstein (*Nature* (1975) 256:495-497) as well as other techniques such as the human B-cell hybridoma technique (Kozbar et al.*, Immunol*. *Today* (1983) 4:72), the EBV-hybridoma technique to produce human monoclonal antibodies (Cole et al. *Monoclonal Antibodies in Cancer Therapy* (1985) (Allen R. Bliss, Inc., pages 77-96), and screening of combinatorial antibody libraries (Huse et al., *Science* (1989) 246:1275). Hybridoma cells can be screened immunochemically for production of antibodies specifically reactive with the protein or peptide and monoclonal antibodies isolated.

The term antibody as used herein is intended to include fragments thereof which are also specifically reactive with gp39 protein or peptide thereof or gp39 fusion protein. Antibodies can be fragmented using conventional techniques and the fragments screened for utility in the same manner as described above for whole antibodies. For example, F(ab')₂ fragments can be generated by treating antibody with pepsin. The resulting F(ab')₂ fragment can be treated to reduce disulfide bridges to produce Fab' fragments. The antibody of the present invention is further intended to include bispecific and chimeric molecules having an anti-gp39 portion.

When antibodies produced in non-human subjects are used therapeutically in humans, they are recognized to varying degrees as foreign and an immune response may be generated in the patient. One approach for minimizing or eliminating this problem, which is preferable to general immunosuppression, is to produce chimeric antibody derivatives, i.e., antibody molecules that combine a non-human animal variable region and a human constant region. Chimeric antibody molecules can include, for example, the antigen binding domain from an antibody of a mouse, rat, or other species, with human constant regions. A variety of approaches for making chimeric antibodies have been described and can be used to make chimeric antibodies containing the immunoglobulin variable region which recognizes gp39. See, for example, Morrison et al., *Proc. Natl. Acad Sci. U.S.A*. 81:6851 (1985); Takeda et al., *Nature* 314:452 (1985), Cabilly et al., U.S. Patent No. 4,816,567; Boss et al., U.S. Patent No. 4,816,397; Tanaguchi et al., European Patent Publication EP171496; European Patent Publication 0173494, United Kingdom Patent GB 2177096B. It is expected that such chimeric antibodies would be less immunogenic in a human subject than the corresponding non-chimeric antibody.

For human therapeutic purposes the monoclonal or chimeric antibodies specifically reactive with a gp39 protein or peptide can be further humanized by producing human constant region chimeras in which parts of the variable regions, especially the conserved framework regions of the antigen-binding domain, are of human origin and only the hypervariable regions are of non-human origin. Such altered immunoglobulin molecules may be made by any of several techniques known in the art, (e.g., Teng et al., *Proc. Natl. Acad. Sci. U.S.A*., 80:7308-7312 (1983); Kozbor et al., *Immunology Today*, 4:7279 (1983); Olsson et al., *Meth. Enzymol*., 92:3-16(1982)), and are preferably made according to the teachings of PCT Publication WO92/06193 or EP 0239400. Humanized antibodies can be commercially produced by, for example, Scotgen Limited, 2 Holly Road, Twickenham, Middlesex, Great Britain.

Another method of generating specific antibodies, or antibody fragments, reactive against a gp39 protein or peptide is to screen expression libraries encoding immunoglobulin genes, or portions thereof, expressed in bacteria with a gp39 protein or peptide. For example, complete Fab fragments, V 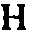 regions and F 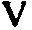 regions can be expressed in bacteria using phage expression libraries. See for example Ward et al., *Nature*, 341: 544-546: (1989); Huse et al., *Science*, 246: 1275-1281 (1989); and McCafferty et al., *Nature*, 348: 552-554 (1990). Screening such libraries with, for example, a gp39 peptide can identify imunoglobin fragments reactive with gp39. Alternatively, the SCID-hu mouse developed by Genpharm can be used to produce antibodies, or fragments thereof.

### B. Soluble Ligands for gp39

Other gp39 antagonists which can be administered to suppress humoral immunity are soluble forms of a gp39 ligand. A monovalent soluble ligand of gp39 can bind gp39, thereby inhibiting the interaction of gp39 with CD40 on B cells. The term soluble indicates that the ligand is not permanently associated with a cell membrane. A soluble gp39 ligand can be prepared by chemical synthesis, or, preferably by recombinant DNA techniques. A preferred soluble gp39 ligand is soluble CD40. Alternatively, soluble gp39 ligand can be in the form of a fusion protein. Such a fusion protein comprises at a least a portion of the gp39 ligand attached to a second molecule. For example, CD40 can be expressed as a fusion protein with immunoglobulin (CD40Ig). In one embodiment, a fusion protein is produced comprising a amino acid residues of an extracellular domain portion of the CD40 joined to amino acid residues of a sequence corresponding to the hinge, CH2 and CH3 regions of Cγ1 to form a CD40Ig fusion protein (see e.g., Linsley et al. (1991) *J. Exp. Med*. 1783:721-730; Capon et al. (1989) *Nature* 337, 525-531; and Capon U.S. 5,116,964). The fusion protein can be produced by chemical synthesis, or, preferably by recombinant DNA techniques based on the cDNA of CD40 (Stamenkovic et al., *EMBO J*., 8:1403-1410(1989)).

### II. ANTIGENS AGAINST WHICH HUMORAL IMMUNITY IS SUPPRESSED

The invention is directed to suppressing humoral immunity against antigens which require contact-dependent helper functions delivered by Th cells. Antigens classically described as thymus-dependent (TD) antigens are encompassed by the invention. The necessity for contact-dependent "help" from Th cells can be due to the necessity for an interaction between gp39 on T cells and CD40 on B cells. As defined by the current invention, the term "TD antigen" is intended to encompass antigens which require a gp39-CD40 interaction between T cells and B cells for induction of a humoral immune response against the antigen. In general, protein antigens are TD antigens. Another form of TD antigen encompassed by the invention is a molecule, referred to as a hapten, linked to a protein. In this case, the protein acts as a carrier for inducing T cell help in order to induce humoral immune responses against the hapten.

The TD antigen of the invention can be administered in soluble form to a subject, e.g., injection of a soluble protein, or the TD antigen can be on the surface of a cell, e.g., a cell-surface protein. The TD antigen can be administered to a subject with a gp39 antagonist or a subject may be exposed to a TD antigen environmentally, for example an allergen. In preferred embodiments, the TD antigen is an agent administered to a subject for therapeutic purposes. This agent can be, for example, a therapeutic antibody or other form of therapeutic drug which is a TD antigen. Inhibiting a humoral immune response against, for instance, a therapeutic antibody, can prolong its efficacy *in vivo* by preventing clearance of the therapeutic antibody in a subject. Small molecules acting as therapeutic agents can also be target antigens against which a humoral response is suppressed if these molecules (functioning as haptens) are administered with a protein or other carrier that induces T cell helper function to activate B cells; suppressing humoral immunity against these therapeutic agents can likewise prolong their effectiveness.

The methods of the invention provide for suppression of humoral immunity against TD antigens while not affecting responses to thymus-independent type II (TI-2) antigens. TI-2 antigens include polysaccharides and lipids which can non-specifically activate B cells in a polyclonal manner. As defined by the current invention, the term "TI-2 antigen" is intended to encompass all antigens which do not require a gp39-CD40 interaction between T cells and B cells for induction of a humoral immune response against the antigen. The current invention provides a method for identifying whether an antigen is a TD antigen or a TI-2 antigen, as defined in the invention, by determining whether humoral immune responses to the antigen can be inhibited by a gp39 antagonist.

### III. SUPPRESSION OF HUMORAL IMMUNITY

The invention pertains to methods of inhibiting a humoral immune response against a TD antigen. The humoral immune response can be a primary immune response, in the case of a first exposure to a TD antigen, or the response can be a secondary humoral immune response, in the case of reexposure to the antigen. Production of one or more isotypes of antibodies can be inhibited. For a primary humoral immune response, in which IgM is the predominant antibody produced, IgM production is predominantly suppressed. For secondary immune response, production of several different isotypes, incuding IgM, IgG and IgE can be suppressed.

The invention provides methods for prolonged suppression of humoral immunity against a TD antigen. As used herein, "prolonged suppression" means that suppression of antibody production against a TD antigen is maintained after administration of a gp39 antagonist *in vivo* has been terminated.

### IV. ADMINISTRATION OF GP39 ANTAGONISTS

Humoral immune responses to a TD antigen can be inhibited according to the methods described herein by administration of a gp39 antagonist to a subject which is exposed to the TD antigen. In one embodiment, the gp39 antagonist is administered in conjunction with the TD antigen. The gp39 antagonist is preferably administered simultaneously with the TD antigen, but can be administered prior to administering the TD antigen or subsequent to the TD antigen, as long as the gp39 antagonist is administered before the TD antigen has induced B cell activation. In other embodiments, a subject is exposed to an antigen environmentally. In this case, a gp39 antagonist should be administered *in vivo* following exposure to the antigen, in close enough in time to prevent B cell activation.

The antagonists of the invention are administered to subjects in a biologically compatible form suitable for pharmaceutical administration *in vivo* to suppress humoral immune responses. By "biologically compatible form suitable for administration *in vivo*" is meant a form of the antagonist to be administered in which any toxic effects are outweighed by the therapeutic effects of the protein. The term subject is intended to include living organisms in which an immune response can be elicited, e.g., mammals. Examples of subjects include humans, dogs, cats, mice, rats, and transgenic species thereof. Administration of an antagonist which interferes with the interaction of gp39 and CD40 as described herein can be in any pharmacological form and a pharmaceutically acceptable carrier. Administration of a therapeutically active amount of the therapeutic compositions of the present invention is defined as an amount effective, at dosages and for periods of time necessay to achieve the desired result. For example, a therapeutically active amount of an antagonist which interferes with the interaction of gp39 and CD40 may vary according to factors such as the disease state, age, sex, and weight of the individual, and the ability of the antagonist to elicit a desired response in the individual. Dosage regima may be adjusted to provide the optimum therapeutic response. For example, several divided doses may be administered daily or the dose may be proportionally reduced as indicated by the exigencies of the therapeutic situation.

The active compound (e.g., antagonist) may be administered in a convenient manner such as by injection (subcutaneous, intravenous, etc.), oral administration, inhalation, transdermal application, or rectal administration. Depending on the route of administration, the active compound may be coated in a material to protect the compound from the action of enzymes, acids and other natural conditions which may inactivate the compound.

To administer an antagonist which interferes with the interaction of gp39 and CD40 by other than parenteral administration, it may be necessary to coat the antagonist with, or co-administer the antagonist with, a material to prevent its inactivation. For example, an antagonist can be administered to an individual in an appropriate carrier or diluent, co-administered with enzyme inhibitors or in an appropriate carrier such as liposomes. Pharmaceutically acceptable diluents include saline and aqueous buffer solutions. Enzyme inhibitors include pancreatic trypsin inhibitor, diisopropylfluorophosphate (DEP) and trasylol. Liposomes include water-in-oil-in-water emulsions as well as conventional liposomes (Strejan et al., (1984) *J. Neuroimmunol* 7:27).

The active compound may also be administered parenterally or intraperitoneally. Dispersions can also be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, asorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating active compound (e.g., antagonist which interferes with the interaction of gp39 and CD40) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (e.g., antagonist) plus any additional desired ingredient from a previously sterile-filtered solution thereof.

When the active compound is suitably protected, as described above, the protein may be orally administered, for example, with an inert diluent or an assimilable edible carrier. As used herein "pharmaceutically acceptable carrier" includes any and all solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the active compound, use thereof in the therapeutic compositions is contemplated. Supplementary active compounds can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

### V. COADMINISTRATION OF GP39 ANTAGONISTS AND OTHER IMMUNOSUPPRESSIVE AGENTS

It has been shown that the interference by soluble CTLA-4 of CD28 triggering, a costimulatory molecule on Tₕ cells, also suppresses TD antibody responses 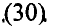
and blocks xenogeneic graft rejection 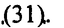 Similar to anti-gp39 administration, soluble CTLA-4 induced a state of prolonged immune suppression. Because anti-gp39 and CTLA-4 mediate their immunosuppressive effects at distinct stages of the humoral immune response, coadministration of these two immunosuppressive drugs may provide additive or synergistic immunosuppressive effects on immunity.

Allergic responses are mediated by IgE antibodies. The production of IgE responses requires the cytokine IL-4. Inhibition of IgE responses against a TD antigen may be more efficient by coadministration of a gp39 antagonist and an inhibitor of IL-4, for example an anti-IL-4 antibody.

This invention is further illustrated by the following examples which should not be construed as limiting. The contents of all references and published patent applications cited throughout this application are hereby incorporated by reference. The contents of a patent application filed on even date herewith in the name of Randolph J. Noelle et al. and entitled "Methods for Inducing Antigen-Specific T Cell Tolerance" is incorported herein by reference.

The following methodology was used in the examples.

### Materials and Methods

Animals. Female, 6-8 week old Balb/c mice (Jackson Laboratories, Bar Harbor, ME) were used for the *in vivo* experiments presented in this study. Animals were maintained in the specific pathogen-free animal facility at Dartmouth Medical School.
Helper T cell clones (Tₕ1). D1.6, an I-A^{d}-restricted, rabbit Ig-specific Tₕ1 clone 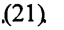 was obtained from Dr. David Parker, University of Mass. at Worcester. In this paper, D1.6 will be referred to as Tₕ1.
Reagents and Antibodies. MR1, hamster anti-murine gp39 mAb 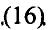 was purified by DEAE HPLC from ascites fluid. Hamster Ig (HIg), used as a control antibody, was purified similarly from hamster serum (Accurate Chemical ad Scientific Corp., Westbuty, NY). RG7/7.6.HL, a mouse anti-rat κ chain (strongly crossreactive with hamster κ chain) antibody, (RG7), 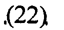 was conjugated with HRPO or FITC and used as a secondary reagent to detect MR1 and HIg. Affinity-purified Goat anti-mouse IgM, IgG₁, IgG₂ₐ, IgG_{2b} and IgG₃ (Southern Biotechnology, Birmingham Al) were used as detection antibodies in the antigen specific ELISAs as well as in the total IgM and IgG₁ ELISAs. B1E3, (kindly provided by Dr. T. Waldschmidt, Univ. of Iowa) a monoclonal anti-murine IgE, was used as the detection antibody for the IgE anti-KLH ELISA. Chimeric-L6 (Chi-L6), a humanized IgG₁ specific for the tumor antigen L6 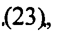 was kindly provided by Bristol-Myers Squibb Pharmaceutical Research Institute, Seattle WA. Anti-CD4, GK 1.5 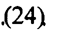 was prepared by HPLC purification of ascites fluid. Sheep red blood cells (SRBC) were purchased from Colorado Serum Co. (Denver, CO). Sea Plaque agarose for use in anti-SRBC plaque assay was obtained from FMC Corporation (Rockland MA). Baby rabbit complement was purchased from Cedarlane (Hornby, Ontario Canada). KLH, Keyhole limpet hemocyanin, (from *Megathura crenulata*) was purchased from Calbiochem (LaJolla CA). Complete Freund's adjuvant (CFA) for immunizations was obtained from Sigma Chemical Co (St. Louis, MO). TNP-SRBC, TNP-KLH and TNP-BSA were prepared as previously described 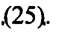
Immunizations for Generation of In Vivo Primary and Secondary Antibody Responses. *Primary Immune Response*s. For eliciting primary antibody responses to SRBC or TNP-SRBC, mice were immunized with 200 µl of 1% SRBC or TNP-SRBC suspension (i.v.). The IgM, anti-SRBC response was assayed 5d after administration of antigen using a modification of the Jerne plaque assay 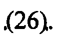 IgM anti-TNP responses were measured by ELISA on day 6. Primary responses to the heterologous immunoglobulin Chi-L6 were generated by i.p. immunization of 100 µg Chi-L6 on alum per mouse. The serum IgM anti-Chi-L6 antibody response was measured after 7d. Primary responses to TNP-Ficoll were generated by immunization with 25 µg of TNP-Ficoll i.p. The IgM anti-TNP response was measured on day 6 by ELISA.
   *Secondary Immune Responses*. For generation of secondary humoral responses to KLH, animals were immunized with KLH in CFA (50 µg; i.p.). Mice were subsequently challenged with 10 µg of soluble KLH (i.p.) three months later. The anti-KLH antibody response was measured on d7 from the serum of immune mice utilizing isotype specific ELISAs. Secondary antibody responses to Chi-L6 were generated by challenging Chi-L6 immune mice with 10 µg soluble Chi-L6, i.p. The serum IgG₁ anti-Chi-L6 antibody response was measured after 7d.
Anti-gp39 Treatment. Sterile, HPLC-purified anti-gp39 (MR1) or HIg (as an antibody control) was administered (i.p.) on d0, d2, d4 post immunization or challenge as indicated for each experiment.
Antigen Specific ELISAs. The antigen specific IgM, IgG₁, IgG₂ₐ, IgG_{2b}, IgG₃, and IgE antibody titers were determined using isotype specific ELISAs. Briefly, antigen, (1 mg/ml of KLH, Chi-L6, TNP₁₆-BSA, or TNP₂-BSA in PBS) was absorbed onto flexible polyvinyl microtiter dishes, overnight at 4°C. Plates were washed and blocked with PBS-1% FCS-sodium-azide. Diluted serum samples were incubated for 2 hours at 37°C. Samples were washed and the antigen specific antibody titers determined with one of the following alkaline-phosphatase conjugated detection antibodies: goat anti-mouse IgM, IgG₁, IgG₂ₐ, IgG_{2b}, or IgG₃ (Southern Biotechnology, Birmingham, Al). The IgE specific ELISA was detected using biotin-conjugated B1E3 followed by alkaline-phosphatase avidin (South San Francisco, CA). All ELISAs were developed by reaction of alkaline-phosphatase with phosphatase substrate (Sigma Chemical, Co., St. Louis, MO). Plates were analyzed on a Dynatech MR700 ELISA reader at 410 nm. Units represent arbitrary values based on the titration curve of a standard immune serum. All experimental groups were titered from 1:100 to 1:100,000 and the titer ascertained based on multiple point analysis. The levels of anti-KLH, anti-Chi-L6 and anti-TNP antibodies in unchallenged controls were below detection.
Detection of Serum Anti-gp39.
   *Quantitation of intact ant-gp39 in the serum of anti-gp39-treated mice:* Serum from mice receiving 750 µg anti-gp39 (250 µg on d0, d2, d4) was obtained on d7, d14, and d21 after initiation of anti-gp39 treatment. The serum was run on a 7.5% SDS gel under non-reducing conditions, transferred to nitrocellulose, and blotted with HRPO-conjugated RG7. Following chemiluminescent detection, areas of the blot corresponding to 150-165 kDa were scanned and digitized using an *Apple Scanner* and the *Image 4.1* software program.
   *Analysis for biologically active ant-gp39 in the serum of treated mice:* Anti-CD3-activated Tₕ1, which express gp39, were stained with dilutions of serum from mice receiving 750 µg anti-gp39 (250 µg on d0, d2, d4) to determine the amount of biologically active gp39 remaining in the serum. Titrations of serum containing anti-gp39 were incubated with activated Tₕ1 cell clones for 30 minutes at 4°C, followed by washing and subsequent incubation with FITC-RG7 for 30 minutes at 4°C. A standard curve of MFI vs anti-gp39 concentration was generated using purified anti-gp39. Samples were analyzed on a Becton Dickinson FACScan and the percent anti-gp39 remaining in the serum was deduced based on the anti-gp39 standard curve. The level of anti-gp39 present in the serum at d7 was set at 100%.
Adoptive Transfer of Helper T cells. Mice were immunized with SRBC (200 µl of 1% SRBC, i.v.) and administered anti-gp39 or HIg (250 µg on d0, d2, d4). On d7 the splenocytes from nonimmune or SRBC-immune mice were removed, erythrocyte depleted, washed and transferred (i.v., 50 x 10⁶/ mouse) into irradiated recipients (600 rads) with or without 50 x 10⁶ spleen cells from TNP-KLH primed (TNP-KLH-CFA, 50 µg i.p.) mice as a source of immune B cells. At the time of transfer, mice were immunized with TNP-SRBC (200 µl of 1% TNP-SRBC iv.) Serum IgG₁ anti-TNP titers were ascertained on d6 post-transfer.

### EXAMPLE 1: Anti-gp39 Inhibits the Generation of Primary Antibody Responses to Erythrocyte Antigens

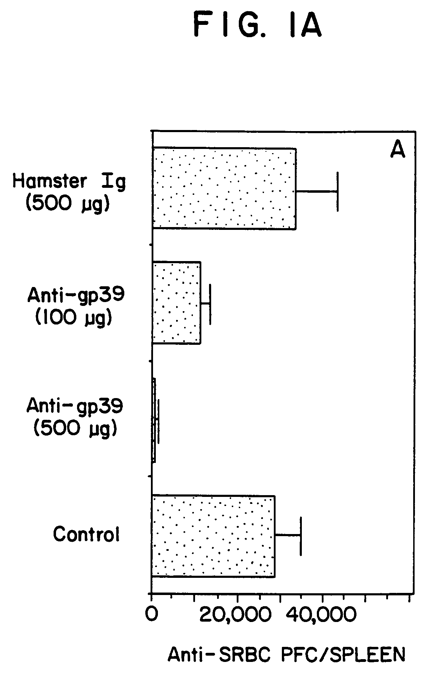

The impaired TD immunity observed in patients with HIM, as well as the potent inhibitory effects of anti-gp39 and CD40-Ig on Tₕ-dependent B cell activation *in vitro*, provided the basis for the study of the potential immunosuppressive effects of anti-gp39 on humoral-mediated immunity *in vivo*. To investigate the role of gp39-CD40 interactions in primary TD humoral immune responses, the effect of *in vivo* administration of anti-gp39 on the primary antibody response to sheep red blood cells (SRBC) was determined. Animals were immunized with SRBC and administered anti-gp39 mAb (or control HIg) over the course of 4d. On d5, the primary anti-SRBC antibody response of anti-gp39-treated, HIg-treated, and control mice was ascertained The IgM anti-SRBC plaque-forming cell (PFC) response of mice that received a total of 1.5 mg of anti-gp39 (500 µg/mouse on d0, d2 and d4) was reduced 99% when compared to the anti-SRBC PFC response from control or HIg-treated mice (Figure 1A). In addition, administration of as little as 300 µg/mouse (100 µg/mouse on d0, d2, and d4) of anti-gp39 reduced the anti-SRBC primary immune response by 66%. Results from these experiments demonstrate that anti-gp39 treatment ablates primary antibody responses *in vivo*.

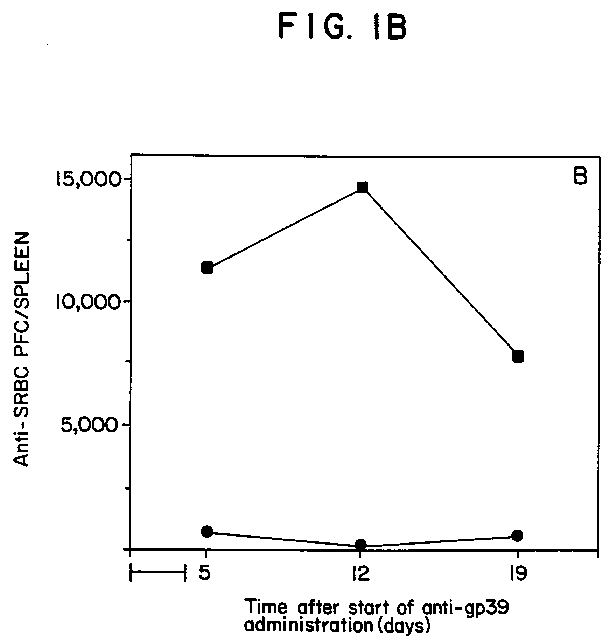

The duration of the immunosuppressive effects of anti-gp39 on the primary humoral immune response to SRBC was subsequently examined. Mice immunized with SRBC were treated with anti-gp39 for 4d and assayed at various later time points for the capacity to mount a primary anti-SRBC response. In this set of experiments, all animals were immunized with SRBC on d0 and administered anti-gp39 or HIg on d0, d2, d4. The IgM anti-SRBC PFC response was measured for one group on d5. Additional SRBC-immune groups were challenged with SRBC on d7 or d14. Five days following the each antigenic challenge (d12 and d19, respectively), the IgM anti-SRBC PFC response was measured. The results of one such experiment are depicted in Figure 1B. As in Figure 1A, the primary anti-SRBC responses were inhibited 80-90% 5d after anti-gp39 administration was begun. In addition, the primary anti-SRBC responses 12d and 19d following anti-gp39 treatment were also inhibited >90%. These results demonstrate that brief anti-gp39 treatment results in prolonged inhibition of primary antibody responses.

### EXAMPLE 2: Anti-gp39 Inhibits the Generation of Secondary Anti-KLH Antibody Responses

Experiments examining primary antibody responses suggest that gp39-CD40 interactions play a critical role in the initiation of primary humoral immunity. However, these experiments do not address the whether gp39-dependent CD40 signalling is required for the generation of secondary antibody responses. Therefore, the effects of anti-gp39 administration on the secondary immune response to soluble challenge with KLH was determined in KLH-immune mice.

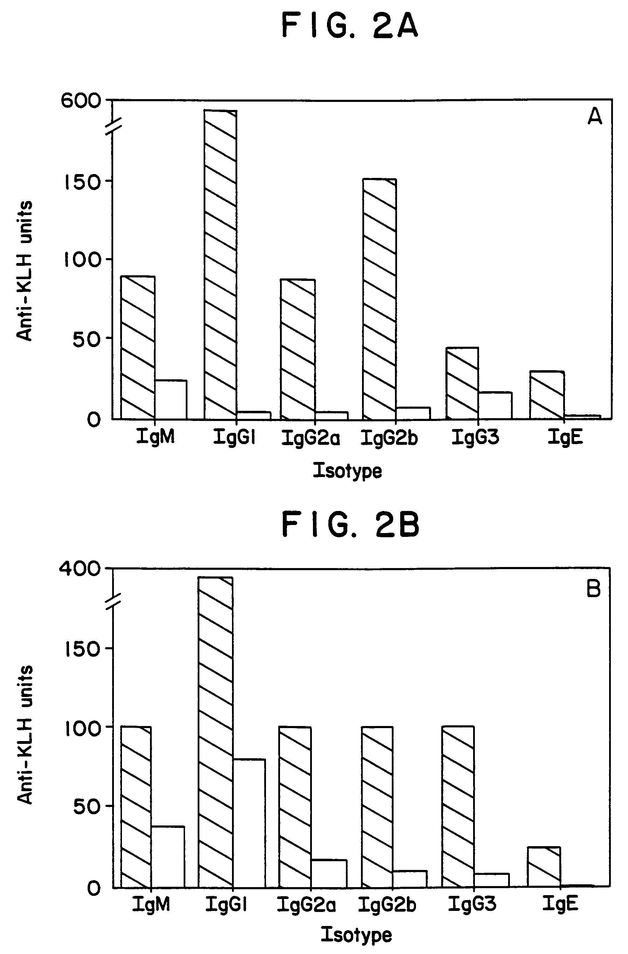

Using schedules of anti-gp39 administration that reduced the primary anti-SRBC PFC response, experiments were designed to evaluate the effects of anti-gp39 treatment on the secondary antibody responses. In these experiments, KLH-immune mice (immunized 3 months prior with CFA and KLH) were challenged with soluble KLH (10 µg/mouse/i.v.). On the day of antigen challenge (d0), mice were also given 250 µg of anti-gp39 or HIg, followed by anti-gp39 or HIg on d2 and d4. At d7 (Fig. 2 panel A) and d14 (Fig. 2 panel B) following challenge with KLH, the mice were bled and the titers of IgM, IgG₁, IgG₂ₐ, IgG_{2b}, IgG₃ and IgE anti-KLH antibodies were determined. The results demonstrate several points: 1) challenge with soluble KLH induced an enduring secondary immune response that persisted for up to 14d; 2) the administration of anti-gp39 significantly reduced the secondary anti-KLH response of the isotypes measured when compared to the administration of equal quantities of HIg; and 3) the immunosuppressive effects of anti-gp39 appeared to be sustained for at least 14d after the initiation of anti-gp39 treatment. Taken together, results from these experiments demonstrate that similar to primary humoral immune responses, the generation of secondary humoral immune responses were also blocked by anti-gp39.

### EXAMPLE 3: Anti-gp39 Inhibits the Generation of Antibody Responses to Heterologous Ig

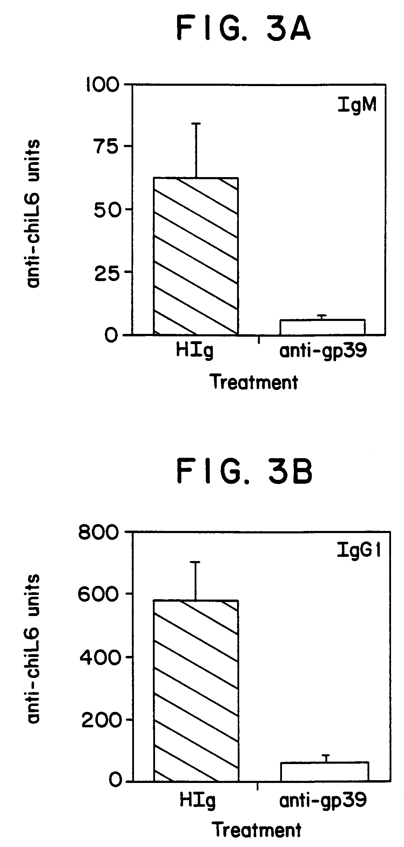

Experiments depicted in Figure 1 demonstrate the immunosuppressive activity of anti-gp39 during a primary response to a strongly immunogenic particulate antigen, SRBC. The cellular nature of erythrocytes makes them unique in their capacity to elicit strong immune responses. Heterologous Ig molecules share this characteristic of being highly immunogenic, and therefore provide an additional model antigen system with which to examine the effects of anti-gp39 treatment on the generation of primary and secondary antibody responses. Animals were immunized with a heterologous Ig molecule, Chi-L6, a humanized mouse anti-tumor cell mAb, and treated with anti-gp39 or control HIg. After 7d, sera was collected and assayed for the production of IgM anti-Chi-L6 antibodies In addition, mice were challenged with Chi-L6 14d after initial immunization and anti-gp39 treatment, and assayed for IgG₁ anti-Chi-L6 antibody production on d21. Figure 3 depicts the results of one such experiment. The primary antibody response to Chl-L6 in mice treated with anti-gp39 is inhibited by > 90% when compared to HIg-treated mice. Moreover, the secondary, IgG1 response to Chi-L6 is similarly inhibited. These results demonstrate that anti-gp39 treatment ablates primary and secondary antibody responses to a second type of TD antigen, heterologous Ig, as effectively as it suppresses responses to erythrocyte and soluble protein antigens.

### EXAMPLE 4: Anti-gp39 Does Not Inhibit the Generation of Primary Antibody Responses to the T-Independent Type II Antigen, TNP-Ficoll

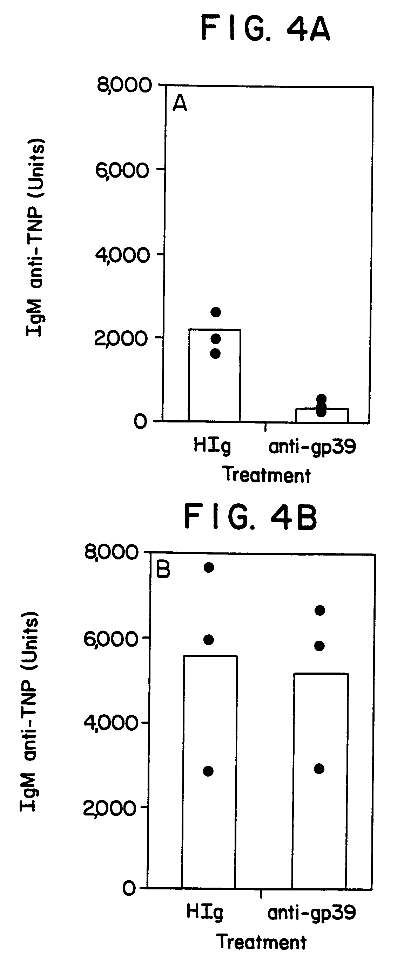

Although the previous experiments demonstrate that anti-gp39 effectively blocks the generation of primary and secondary antibody responses to TD antigens *in vivo*, it is unclear whether gp39-CD40 interactions play a role in the initiation of humoral responses to TI antigens. Data presented in the accompanying paper demonstrate that immunization with the TI-type II antigen, TNP-Ficoll, results in gp39 expression by Tₕ cells *in vivo*. In order to address whether gp39-CD40 interactions are necessary for the generation of antibody responses to this TI antigen, the affect of anti-gp39 treatment on mice immunized TNP-Ficoll, was assessed. Mice immunized with TNP-Ficoll or TNP-SRBC were treated with anti-gp39 or HIg and the IgM anti-TNP antibody response determined after 6 days. Figure 4A demonstrates that animals immunized with the TD antigen TNP-SRBC elicit significant anti-TNP serum antibody responses. As predicted from the previously described experiments, anti-gp39 treatment dramatically inhibits the primary anti-TNP response generated in these mice. In contract, mice immunized with TNP-Ficoll mount a higher titered anti-TNP antibody response (Figure 4B); however, treatment with anti-gp39 does not inhibit the antibody response to TNP-Ficoll. Results from these experiments demonstrate that, unlike responses to TD antigens, anti-gp39 does not block the generation of humoral responses to TNP-Ficoll, suggesting that responses to TI antigens may be gp39-independent.

### EXAMPLE 5: Anti-gp39 Administration Does Not Functionally Delete SRBC-Specific Tₕ

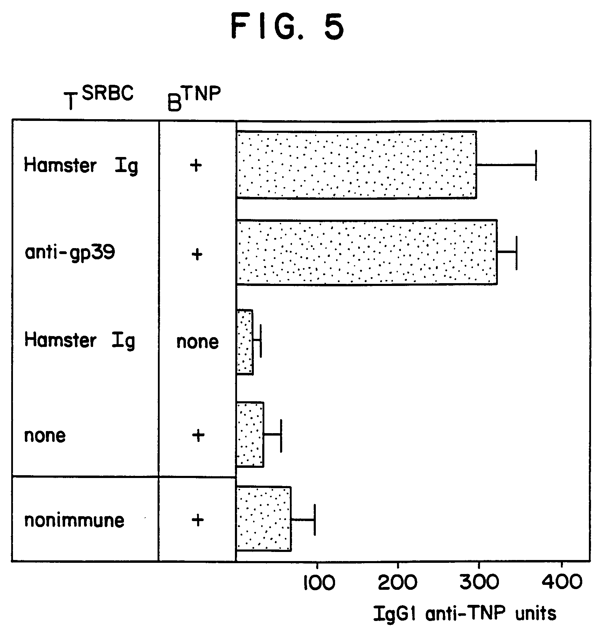

From the previous experiments, it is known that anti-gp39 interferes with the development of TD humoral immunity; however, the mechanism by which anti-gp39 treatment suppresses humoral responses is not clear. Immune suppression by anti-gp39 could be mediated by: 1) the negative signalling of gp39-bearing T cells causing Tₕ anergy; 2) mAb-mediated cytotoxic deletion of anti-gp39 bearing CD4⁺ T cells; and/or 3) the blocking of gp39 binding to CD40. A series of experiments were performed to gain insight into which of these mechanisms may be operative in the protracted immune suppression observed with anti-gp39 therapy. To explore the possibility that antigen-specific Tₕ were deleted or anergized by anti-gp39 therapy, antigen-specific Tₕ function from gp39-treated mice was measured by adoptive transfer. Briefly, mice were immunized with SRBC (to prime SRBC-specific Tₕ) and administered anti-gp39 or HIg (250 µg/mouse on d0, d2, d4). After 7d, spleen cells from unimmunized mice or SRBC-immune spleen cells from HIg-treated or anti-gp39-treated mice were adoptively transferred into recipient mice with TNP-immune spleen cells as a source of TNP-primed B cells. Mice were simultaneously challenged with TNP-SRBC, and the IgG₁ anti-TNP titer ascertained on d5. SRBC-primed T helper cells are required to elicit a secondary anti-TNP response in the recipient mice as demonstrated by the fact that recipients which received spleen cells from nonimmune donors produced substantially lower IgG1 anti-TNP compared to those mice which received spleen cells from SRBC-primed animals (Figure 5). More importantly, results of these experiments revealed that the SRBC helper activity from HIg-treated and anti-gp39-treated mice was similar, indicating that anti-gp39 treatment did not alter Tₕ function or block the priming of Tₕ. Moreover, antigen-responsive Tₕ were not deleted or anergized as a result of anti-gp39 treatment, as they provided helper-effector function upon transfer.

### EXAMPLE 6: In Vivo Clearance of Hamster Anti-gp39

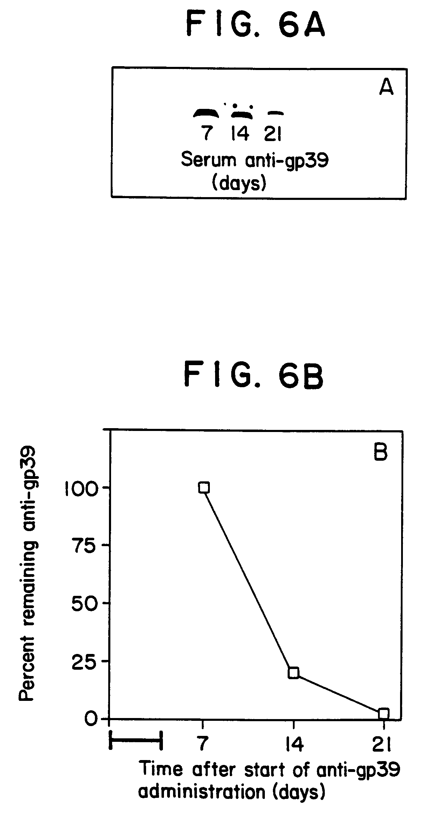

Previous studies have established that anti-gp39 (MR1) blocks the binding of gp39 to CD40 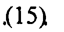 and thus support the hypothesis that the *in vivo* immunosuppressive effects of anti-gp39 are due to the blocking of gp39-CD40 interactions. Assuming this hypothesis correct, the long-term immune suppression observed with anti-gp39 administration requires the persistence of anti-gp39 in the host. To determine if anti-gp39 could be detected for the period of time that immune suppression was evident, the *in vivo* clearance rate of anti-gp39 from serum was determined. Mice were given a regime of antibody (3 x 250 µg anti-gp39) over the course of 4d and assayed for the levels of serum anti-gp39 at 7d, 14d, and 21d after the initiation of antibody administration. Western blot analysis for non-reduced MR1 (160 kd) indicated that intact, serum anti-gp39 could be detected for at least 21d after the initiation of antibody treatment (Figure 6A). The serum concentration of anti-gp39 in animals at 21d was approximately 5% (based on scanning densitometry), when compared to the signals derived from serum of animals analyzed 7d after initiation of antibody therapy.

Although it was determined that intact anti-gp39 was present in serum, it was also important to ascertain that the anti-gp39 was biologically active. Therefore, sera from mice which received 3 x 250 µg of anti-gp39 over the course of 4d were used to stain gp39-bearing Tₕ (Figure 6B). The level of serum anti-gp39 3d after the last injection (7d after initiation of antibody treatment) was set at 100%. Fourteen days after the initiation of antibody therapy, approximately 10-15% of the biologically active anti-gp39 mAb was detected in the serum. Twenty-one days post-initiation of therapy, 2-3% of anti-gp39 remained in the serum. Therefore, both the determination of intact gp39 by Western blotting and of biologically active anti-gp39 revealed that approximately 5% of the anti-gp39 was present 21d after beginning anti-gp39 therapy. These results demonstrate the half-life of anti-gp39 to be approximately 12d and offer evidence consistent with the hypothesis that prolonged suppression of humoral immune responses by anti-gp39 is due to persistent blocking of Tₕ function.

The present study demonstrates that *in vivo* administration of an anti-gp39 antibody which blocks gp39-CD40 interactions *in vitro*, results in profound inhibition of both primary and secondary humoral immune responses to TD antigens, but not TI-type II antigens. In addition, this study demonstrates that anti-gp39 treatment does not block the priming of antigen-primed Tₕ cells. Therefore, the gp39-CD40 ligand-receptor pair can be used as a target for the therapeutic manipulation of the humoral immune response.

To gain insight into how anti-gp39 was exerting its immunosuppressive effect on humoral immunity, the direct effects of anti-gp39 on Tₕ function were addressed. The data indicate that SRBC-immune Tₕ from anti-gp39-treated mice were fully capable of providing help upon adoptive transfer, suggesting that anti-gp39 treatment did not cause Tₕ deletion or anergy *in vivo*. These results led to the speculation that anti-gp39 mediates its immunosuppressive effects by blocking gp39 binding to CD40 and not by the inactivation of gp39-bearing Tₕ. In support of this hypothesis *in vitro* studies have established that anti-gp39 blocks the binding of CD40 to gp39 (16). Furthermore, biologically active anti-gp39 could be detected in serum for the period of time that immune suppression was apparent. Although only 5% of anti-gp39 was present in serum at a time when immune suppression was evident, it is possible that the local tissue concentrations of anti-gp39 in specific sites of secondary lymphoid organs is higher and clearance rates are slower than that of serum anti-gp39. Treatment of mice with anti-gp39 inhibited the primary immune response to SRBC and heterologous Ig >90% for prolonged periods of time. Assuming that anti-gp39 is mediating the inhibition by blocking gp39 function, these data implicate gp39-CD40 interactions as essential in the development of primary immune responses to TD antigens. Immunohistochemical analysis establish that gp39 is induced as a consequence of immunization with TD antigens and may be of functional significance. The *in situ* studies of gp39 expression illustrate that the initial site of gp39-CD40 interactions during primary humoral immune responses is in the peripheral aspects of the periarteriolar lymphoid sheaths (PALS) and around the terminal arterioles (TA) of the spleen. It is at these sites that conjugates between gp39-expressing Tₕ and antigen-specific B cells were found juxtaposed, suggesting that the outer PALS is a major site of T cell-B cell interactions during primary humoral immune responses. Therefore, the PALS may be the site at which anti-gp39 interacts with gp39-expressing Tₕ cells to ultimately inhibit T-B interaction and subsequent Ig production.

Similar to primary responses, the secondary humoral immune response of mice primed to KLH in CFA was also shown to be inhibited by the administration of anti-gp39. Consistent with the reduction of anti-SRBC PFC by anti-gp39, reductions in serum antibodies titers to antigenic challenge were also observed. The serum titers of all anti-KLH Ig isotypes measured (IgM, IgG₁, IgG₂ₐ, IgG_{2b}, IgG₃, and IgE) were reduced by the treatment of mice with anti-gp39. The effect of anti-gp39 administration was apparent for at least 14d after secondary challenge with antigen, establishing a persistent immune suppression by anti-gp39. Anti-gp39-mediated immune suppression of secondary responses to KLH is not unique to KLH, since secondary immune responses to heterologous Ig and heterologous erythrocytes were also inhibited by anti-gp39 therapy. The anatomical distribution of gp39-expressing Tₕ was identical to that observed upon primary immunization, however, the frequency of gp39-expressing Tₕ in immune spleen was increased over that observed during primary immune responses. No gp39-expressing Tₕ were found in the germinal centers or follicles of immune spleen. Thus, it appears that B cells are triggered to respond to activated Tₕ cells in the PALS and TA of the spleen and later migrate to the follicles and germinal centers.

The focus of the present study was to demonstrate the potential use of anti-gp39 in the control of TD humoral immunity. Brief treatment regimes with anti-gp39 resulted in prolonged suppression, an attractive attribute of this therapeutic antibody. Of special interest may be the capacity of anti-gp39 to prevent primary and secondary humoral responses to other heterologous, therapeutic antibodies such as Chi-L6. This would permit the exposure of patients to repeated administrations of heterologous therapeutic antibodies.

### EXAMPLE 7: Production and Characterization of Anti-gp39 Antibodies

### Experiment 1 - Antibodies directed against human gp39

For induction of antigen-specific T cell tolerance in a human subject, it is preferable to administer an antibody directed against human gp39. The following methodology was used to produce mouse anti-human gp39 monoclonal antibodies. Balb/c mice were immunized with a soluble gp39 fusion protein, gp39-CD8, in Complete Freund's Adjuvant (CFA). Mice were subsequently challenged 6 weeks later with soluble gp39-CD8 in Incomplete Freund's Adjuvant (IFA). Soluble gp39-CD8 was given in soluble form 4 weeks after secondary immunization. Mice were then boosted with activated human peripheral blood lymphocytes 2 weeks later, followed by a final boost with soluble gp39-CD8 after an additional 2 weeks. Splenocytes were fused with the NS-1 fusion partner on day 4 after final immunization as per standard protocols.

Clones producing anti-human gp39 antibodies were selected based on a multiple screening process. Clones were initially screened by a plate binding assay using gp39-CD8. Positive clones were then screened against a control CD8 fusion protein, CD72-CD8. Clones which scored positive on the CD8-CD72 plate binding assay were eliminated. The remaining clones were subsequently screened on resting and 6 hour activated human peripheral blood lymphocytes (PBL) by flow cytometric analysis. Hybridomas staining activated, but not resting, PBL were considered positive. Finally, the remaining clones were tested for their ability to block the binding of CD40Ig to plate bound gp39.

Approximately 300 clones were initially screened against gp39-CD8 and CD72-CD8 in the plate binding assays. Of those clones, 30 were found to detect plate-bound gp39 and not CD8. These clones were subsequently screened for detection of gp39 on activated human PBL. Approximately 15 clones detected a molecule on activated PBL, but not resting cells. Specificity was further confirmed by determining the capacity of the clones to block CD40Ig detection of plate-bound gp39. 3 of 10 clones tested block CD40Ig binding in this assay. These clones were 3E4, 2H5 and 2H8. Such clones are preferred for use in the methods described herein. Clones which tested positive on activated, but not resting PBL, were also screened for reactivity with an activated rat T cell clone, POMC8. The clone 2H8 expressed crossreactivity with this rat T cell line.

### Experiment 2 - Antibodies directed against human gp39

A similar immunization procedure to that described in Experiment 1 was used to produce additional antibodies directed against human gp39. One Balb/c mouse was immunized with soluble gp39-CD8 in CFA, followed by challenge with 6 hour activated human peripheral blood lymphocytes 4 weeks later. The mouse was subsequently boosted with soluble gp39-CD8 4 days prior to fusion of splenocytes with the NS-1 fusion partner per standard protocols. Screening of hybridoma clones was performed by flow cytometric staining of 6 hour activated human PBLs. Clones staining activated but not resting human PBLs were selected. Six clones, 4D9-8, 4D9-9, 24-31, 24-43, 89-76 and 89-79, were selected for further analysis.

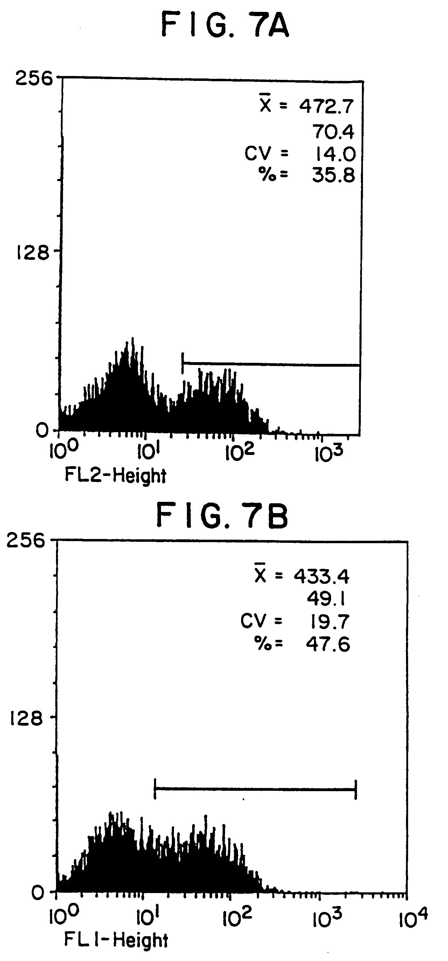

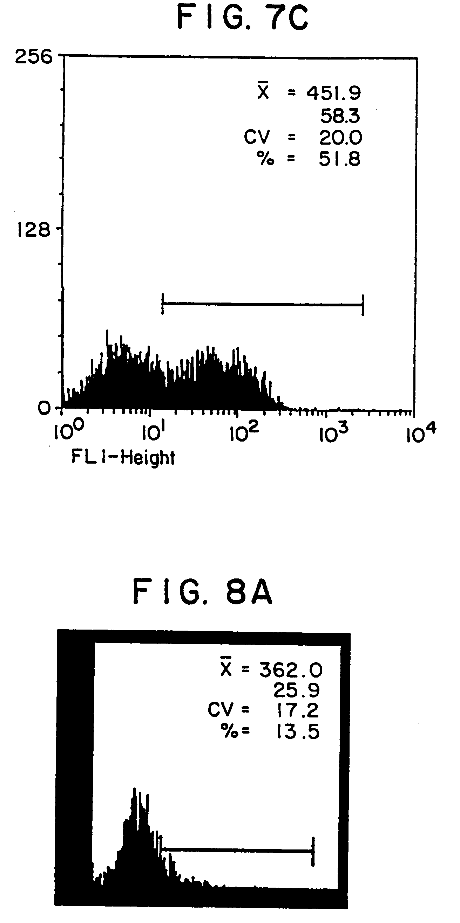

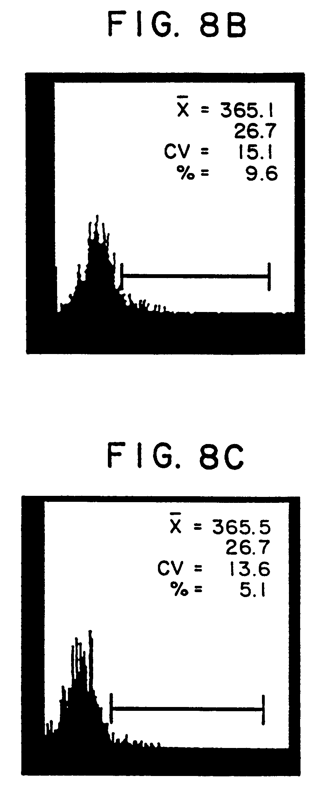

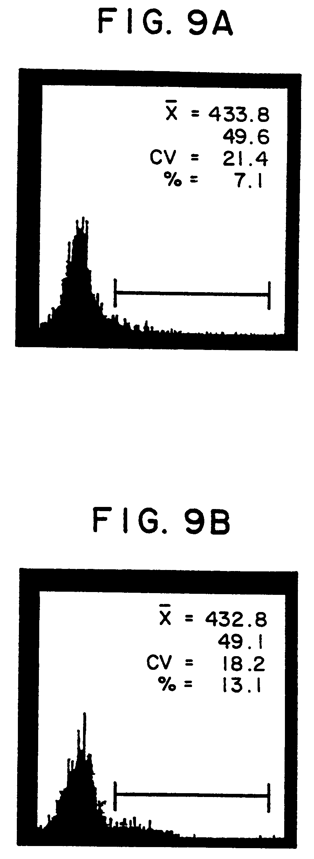

The specificity of the selected antibodies was confirmed by several assays. First, flow cytometric analysis demonstrated that all six mAbs stain activated, but not resting peripheral blood T cells (see Figure 7B and 7C for a representative example, depicting staining of activated T cells with 4D9-8 and 4D9-9, respectively). Expression of the molecule recognized by each of the six antibodies is detectable within 4 hours of activation, is maximal between 6-8 hours after activation, and is undetectable by 24 hours after activation. All six mAbs recognize a molecule expressed on activated CD3⁺ PBLs, predominantly of the CD4⁺ phenotype, but a portion of CD8⁺ T cells also express the molecule. Expression of the molecule recognized by the six mAbs is inhibited by the presence of cyclosporin A in the culture medium, as is the expression of gp39 (see Figure 8A and 8B for a representative example, depicting staining of cyclosporin treated T cells with 4D9-8 and 4D9-9, respectively). The kinetics and distribution of expression of the molecule recognized by these mAbs are identical to that of gp39, as detected by the fusion protein of human CD40Ig. In addition, all six mAbs block the staining of gp39 by CD40Ig (see Figure 9A and 9B for for a representative example, depicting inhibition of gp39 staining by CD40Ig in the presence of 4D9-8 and 4D9-9, respectively). In an ELISA assay, all six mAbs recognize gp39-CD8, a soluble fusion form of the gp39 molecule. Moreover, all six mAbs immunoprecipitate a molecule of approximately 36 kd from ³⁵S-methionine labeled activated human PBLs. The immunoprecipitated molecule is identical to that precipitated by the human CD40Ig fusion protein.

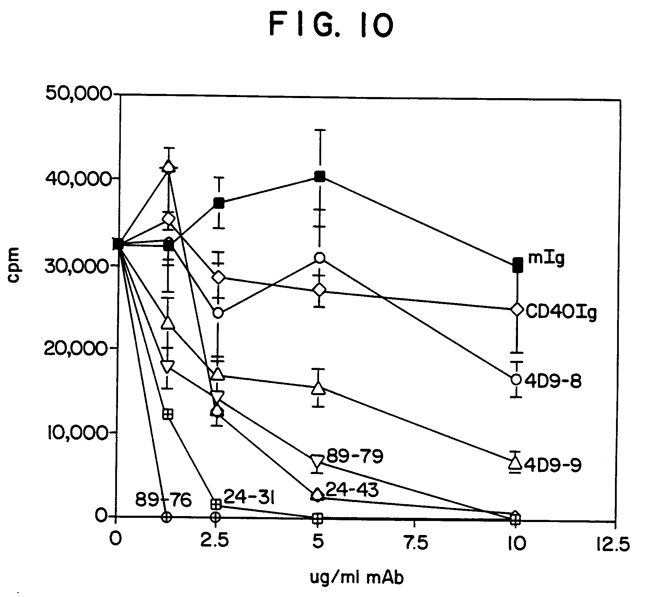

The functional activity of the six selected mAbs (4D9-8, 4D9-9, 24-32, 24-43, 89-76 and 89-79) was assayed as follows. First, the ability of the mAbs to inhibit the proliferation of purified human B cells cultured with IL-4 and soluble gp39 was measured. Purified human B cells were cultured with gp39 and IL-4 in the presence or absence of purified monoclonal antibodies or CD40Ig at dosages between 0 and 12.5 µg/ml. B cell proliferation was determined alter 3 days in culture by thymidine incorporation. The results (shown in Figure 10) demonstrate that all six mAbs can inhibit B cell proliferation induced by gp39 and IL-4. The mAbs 89-76 and 24-31 were most effective at inhibiting the induced B cell proliferation.

Next, the ability of the mAbs to inhibit B cell differentiation, as measured by Ig production induced by anti-CD3 activated T cells and IL-2, was examined. Purified IgD⁺ human B cells were prepared by positive selection with FACS and then cultured with anti-CD3 activated human T cells (mitomycin C treated) and IL-2 for 6 days in the presence or absence of purified anti-gp39 monoclonal antibodies as dosages between 0 and 10 µg/ml. IgM, IgG and IgA production was assessed by ELISA on day 6. The results (shown below in Table 1) demonstrate that all six antibodies can inhibit T cell dependent B cell differentiation, as measured by IgM, IgG and IgA production.

**Table 1**

| | | Production of Immunoglobulin | | |
|---|---|---|---|---|
| mAb | µg/ml | IgM | IgG | IgA |
| none | - | 17,500 | 6710 | 4471 |
| 4D9-8 | 0.1 | 4813 | 2130 | 2819 |
| | 1.0 | 4394 | 2558 | 1519 |
| | 10.0 | 1081 | 389 | 396 |
| 4D9-9 | 0.1 | 3594 | 919 | 1731 |
| | 1.0 | 2659 | 1233 | 1606 |
| | 10.0 | 374 | 448 | 266 |
| 24-31 | 0.1 | 3863 | 981 | 344 |
| | 1.0 | 1287 | 314 | 165 |
| | 10.0 | 1120 | 596 | 23 |
| 24-43 | 0.1 | 6227 | 4132 | 432 |
| | 1.0 | 3193 | 2130 | 192 |
| | 10.0 | 7021 | 1232 | 1081 |
| 89-76 | 0.1 | 3783 | 1069 | 344 |
| | 1.0 | 2180 | 352 | 171 |
| | 10.0 | 818 | 551 | 19 |
| 89-79 | 0.1 | 9763 | 1924 | 3021 |
| | 1.0 | 2314 | 460 | 156 |
| | 10.0 | 183 | 135 | 434 |

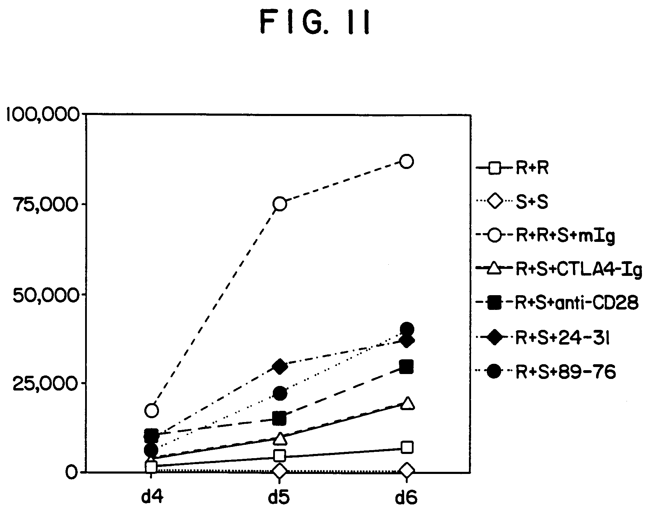

To examine the effect of the anti-gp39 mAbs on T cell responses, the mAbs were included in standard mixed lymphocyte reactions (MLR). 300,000 human peripheral blood lymphocytes (responders = R) were cultured with 100,000 irradiated allogeneic peripheral blood lymphocytes (stimulators = S) in the presence or absence of anti-gp39 mAbs (10 µg/ml). Cultures were pulsed with 3H-thymidine on day 4,5 or 6 and harvested 18 hours later. All six anti-human gp39 mAbs inhibited allo-specific responses as measured by MLR (see Figure 11 for a representative example, depicting inhibition of allo-specific responses when R and S are incubated in the presence of 24-31 or 89-76; a CTLA4-immunoglobulin fusion protein and an anti-CD28 mAb were used as positive controls).

To determine whether the six mAbs recognized distinct epitopes on the human gp39 molecule, crossblocking experiments were performed. Activated human PBLs were first blocked with each of the six mAbs (25 µg/ml of unconjugated antibody). Cells were washed and then stained with 10 µg/ml of biotin-conjugated antibody, followed by reaction with phytoerythrin conjugated avidin (PE-Av). The staining of the cells with PE-Av was analyzed by FACS. The results are shown below in Table 2.

**Table 2**

| Blocking | Staining Antibody | | | | | |
|---|---|---|---|---|---|---|
| Ab | 4D9-8 | 4D9-9 | 24-31 | 24-43 | 89-76 | 89-79 |
| none | +++ | +++ | ++++ | ++++ | ++++ | ++++ |
| 4D9-8 | ND. | - | ++++ | ++++ | +++ | +++ |
| 4D9-9 | +++ | ND | +++ | ++++ | +++ | +++ |
| 24-31 | + | + | ND | +++ | ++ | ++ |
| 24-43 | + | + | +++ | ND | ++ | + |
| 89-76 | + | + | +++ | +++ | ND | +++ |
| 89-79 | + | ++ | +++ | +++ | +++ | ND |
| The intensity of staining and the percentage of positive cells are represented by the + symbol (++++ = MI >200; +++ = MI >125; ++ = MI >50; + = MI >25; - = no staining above background). ND= not determined. | | | | | | |

All antibodies blocked the biding of CD40Ig to activated human PBLs. However, the data shown in Table 2 clearly demonstrate the failure of some antibodies to block the binding of other antibodies to activated human PBLs, suggesting that they recognize distinct epitopes on the human gp39 molecules.

The 89-76 and 24-31 hybridomas, producing the 89-76 and 24-31 antibodies, respectively, were deposited under the provisions of the Budapest Treaty with the American Type Culture Collection, Parklawn Drive, Rockville, Md, on September 2, 1994. The 89-76 hybridoma was assigned ATCC Accession Number HB 11713 and the 24-31 hybridoma was assigned ATCC Accession Number HB 11712.

### Experiment 3- Antibodies directed against mouse gp39

In one embodiment of the invention, the gp39 antagonist is an anti-mouse gp39 monoclonal antibody, MR1. The following method was used to produce the MR1 monoclonal antibody, and may be used to generate other antibodies directed toward gp39.

Hamsters were immunized intraperitoneally with 5-10⁶ activated Tₕ1 cells (d1.6) at weekly intervals for six weeks. When the serum titer against murine Tₕ1 was greater than about 1:10,000, cell fusions were performed with polyethylene glycol using immune hamster splenocytes and NS-1. Supernatant from wells containing growing hybridomas were screened by flow cytometry on resting and activated Tₕ1. One particular hybridoma, which produced a Mab that selectively recognized activated Tₕ was further tested and subcloned to derive MR1. MR1 was produced in ascites and purified by ion exchange HPLC. A hybridoma MR1 has been deposited with the American Type Culture Collection and assigned Accession Number HB11048.

The following references are refered to by number in the examples and detailed description of the invention:
1. Inaba K., M.D. Witmer, R.M. Steinman. 1984. Clustering of dendritic cells, helper T lymphocytes, and B cells during primary antibody responses in vitro. *J Exp. Med*. 160:858.
2. Inaba K., R.M. Steinman. 1985. Protein-specific helper T-lymphocyte formation initiated by dendritic cells. *Science*. 229:475.
3. Noelle R.J., E.C. Snow. 1991. Cognate interactions of helper T cells and B cells. *Immunol. Tod.* 11:361.
4. Gordon J., M.J. Millsum, G.R. Guy, J.A. Ledbetter. 1987. Synergistic interaction between interleukin 4 and anti-Bp50 (CDw40) revealed in a novel B cell restimulation assay. *Eur. J Immunol*. 17:1535.
5. Clark E.A., J.A. Ledbetter. 1986. Activation of human B cells mediated through two distinct cell surface differentiation antigens, Bp35 and Bp 50. *Proc. Natl. Acad. Sci. USA..* 83:4494.
6. Stamenkovic I., E.A. Clark, B. Seed. 1989. A B-lymphocyte activation molecule related to the nerve growth factor receptor and induced by cytokines in carcinomas. *EMBO*. 8:1403.
7. Hollenbaugh D., L. Grosmaire, C.D. Kullas, N.J. Chalupny, R.J. Noelle, I. Stamenkovic, J.A. Ledbetter, A. Aruffo. 1992. The human T cell antigen p39, a member of the TNF gene family, is aligand for the CD40 receptor: Expression of a soluble form of gp39 with B cell co-stimulatory activity. *EMBO*. 11:4313.
8. Armitage R.J., W.C. Fanslow, L. Strockbine, T.A. Sato, K.N. Clifford, B.M. Macduff, D.M. Anderson, S.D. Gimpel, T. Davis-Smith, C.R. Maliszewski, E.A. Clark, C.A. Smith, K.H. Grabstein, D. Cosman, M.K. Spriggs. 1992. Molecular and biological characterization of a murine ligand for CD40. *Nature*. 357:80.
9. Valle A., C.E. Zuber, T. Defrance, O. Djossou, R.M. De, J. Banchereau. 1989. Activation of human B lymphocytes through CD40 and interleukin 4. *Eur. J Immunol*. 19:1463.
10. Gordon J., M.J. Millsum, R.L. Flores, S. Gillis. 1989. Regulation of resting and cycling human B lymphocytes via surface the accessory molecules interleukin-4, CD23 and CD40. *Immunology*. 68:526.
11. Jabara H.H., S.M. Fu, R.S. Geha, D. Vercelli. 1990. CD40 and IgE: synergism between anti-CD40 monoclonal antibody and interleukin 4 in the induction of IgE synthesis by highly purified human B cells. *J Exp. Med*. 172:1861.
12. Banchereau J., F. Rousset. 1991. Growing human B lymphocytes in the CD40 system. *Nature*. 353:678.
13. Armitage R.J., T.A. Sato, B.M. Macduff, K.N. Clifford, A.R. Alpert, C.A. Smith, W.C. Fanslow. 1992. Identification of a source of biologically active CD40 ligand. *Eur. J. Immunol*. 22:2071.
14. Lane P., A. Traunecker, S. Hubele, S. Inui, A. Lanzavecchia, D. Gray. 1992. Activated human T cells express a ligand for the human B cell-associated antigen CD40 which participates in T cell-dependent activation of B lymphocytes. *Eur. J Immunol.* 22:2573.
15. Noelle R.J., J.A. Ledbetter, A. Aruffo. 1992. CD40 and its ligand, an essential ligand-receptor pair for thymus-dependent B cell activation. *Immunol. Tod*. 13:431.
16. Noelle R.J., M. Roy, D.M. Shepherd, I. Stamenkovic, J.A. Ledbetter, A. Aruffo. 1992. A novel ligand on activated T helper cells binds CD40 and transduces the signal for the cognate activation of B cells. *Proc. Natl. Acad. Sci. USA*. 89:6550.
17. Allen R.C., R.J. Armitage, M.E. Conley, H. Rosenblatt, N.A. Jenkins, N.G. Copeland, M.A. Bedell, S. Edelhoff, J. Disteche, D.K. Simoneaux, W.C. Fanslow, J. Belmont, M.K. Spriggs. 1993. CD40 ligand gene defects responsible for X- linked hyper- IgM Syndrome. *Science.* 259:990.
18. Aruffo A., M. Farrington, D. Hollenbaugh, Li X., A. Milatovich, S. Nonoyama, J. Bajorath, L.S. Grosmaire, R. Stenkamp, M. Neubauer, R.L. Roberts, R.J. Noelle, Ledbetter, U. J.A. Francke, H.D. Ochs. 1993. The CD40 ligand, gp39, is defective in activated T cells from patients with X- linked hyper- IgM syndrome. *Cell*. 72:291.
19. DiSanto J.P., J.Y. Bonnefoy, J.F. Gauchat, A. Fischer, G. de Saint Basile. 1993. CD40 ligand mutations in X- linked immunodefiency with hyper- IgM. *Nature*. 361:541.
20. Korthauer U., D. Graf, H.W. Mages, F. Brieres, M. Padayachee, S. Malcolm, A.G. Ugazio, L.D. Notarangelo, R.L. Levinsky, A. Kroczek. 1993. Defective Expression of T- cell CD40 ligand causes X- linked immunodeficiency with hyper- IgM. *Nature.* 361:539.
21. Kurt-Jones E., S. Hamberg, J. Ohara, W.E. Paul, A.K. Abbas. 1987. Heterogeneity of helper/inducer T lymphocytes. I. Lymphokine production. *J Exp. Med*. 166:1774.
22. Springer T.A., A. Bhattacharya, J.T. Cardoza, F. Sanchez-Madrid. 1982. Monoclonal antibodies specific for rat IgG1, IgG2a, and IgG2b subclasses, and kappa chain monotypic and allotypic determinants: reagents for use with rat monoclonal antibodies. *Hybrid*. 1:25.
23. Hellstrom I. 1986. Monoclonal mouse antibodies raised against human lung carcinoma. *Can. Res*. 46:3917.
24. Wilde D.B., P. Marrack, J. Kappler, D.P. Dialynas, F.W. Fitch. 1983. Evidence implicating L3T4 in class II MHC antigen reactivity; monoclonal antibody GK1.5 (anti-L3T4a) blocks class II MHC antigen-specific proliferation, release of lymphokines, and binding by cloned murine helper T lymphocyte lines. *J Immunol*. 131:2178.
25. Snow E.C., R.J. Noelle. 1987. Thymus-dependent antigenic stimulation of hapten-specific B lymphocytes. *Immunol. Rev.* 99:173.
26. Jerne N.K. C. Henry, A. A. Nordin, H. Fuji, A. M. Koros, and I. Lefkovits. 1974. Plaque forming cells: Methodology and theory. *Transplant Rev*. 18:130.
27. Ochs H.D., R.J. Wedgewood. 1989. Disorders of the B cell system. *In* Immunologic disorders in infants and children, Third ed., E.R. Sreihm, ed. (Philadelphia: W. B. Saunders), pp. 226.
28. Notarangelo L.D., M. Duse, A.G. Ugazio. 1992. Immunodeficiency with hyper-IgM (HIM). *Immunodef Rev.* 3:101.
29. Shizuru J.A., S.A. Alters, C.G. Fathman. 1992. Anti-CD4 monoclonal antibodies in therapy: Creation of nonclassical tolerance in the adult. *Immunol. Rev*. 129:103.
30. Linsley P.S., P.M. Wallace, J. Johnson, M.G. Gibson, J.L. Greene, J.A. Ledbetter, C. Singh, M.A. Tepper. 1992. Immunosuppression in vivo by a soluble form of the CTLA-4 T cell activation molecule. *Science*. 257:792.
31. Lenschow D.J., Y. Zeng, J.R. Thistlethwaite, A. Montag, W. Brady, M.G. Gibson, P.S. Linsley, J.A. Bluestone. 1992. Long-term survival of xenogeneic pancreatic islet grafts induced by CTLA41g. *Science.* 257:789.

## Claims

1. A pharmaceutical composition comprising a thus-dependent (TD) antigen and a gp39 antagonist.

2. The pharmaceutical composition of claim 1, wherein the TD antigen is an allergen.

3. The pharmaceutical composition of claim 2 further comprising an IL-4 inhibitor.

4. Use of a gp39 antagonist for the preparation of a medicament for inhibiting a humoral immune response to a thymus-dependent (TD) antigen *in vivo*, wherein the TD antigen is administered to a subject with the antagonist.

5. The use of claim 4, wherein the humoral immune response is a primary humoral immune response or a secondary humoral immune response.

6. The use of claim 4 or 5, wherein humoral immune responses to TI-2 antigens are not inhibited.

7. Use of a gp39 antagonist for the preparation of a medicament for providing for suppression of an antigen specific IgE response to an administered thymus-dependent (TD) antigen *in vivo,* wherein the gp39 antagonist is administered to a subject exposed to the TD antigen, and suppression of antibody production against the administered antigen is maintained after administration of the gp39 antagonist has terminated.

8. Use of a gp39 antagonist for the preparation of a medicament for providing for suppression of a humoral response to an administered allergen to which a subject is exposed, wherein suppression of antibody production against the administered antigen is maintained after administration of the gp39 antagonist has terminated.

9. The use of claim 8, wherein an IL-4 inhibitor is coadministered to the subject.

10. The composition of claim 3 or the use of claim 9, wherein the IL-4 inhibitor is an IL-4 antibody.

11. Use of a gp39 antagonist for the preparation of a medicament for suppression of a humoral immune response to an administered thymus-dependent (TD) antigen *in vivo*, wherein the TD antigen is administered to a subject with the gp39 antagonist, and suppression of antibody production against the administered antigen is maintained after administration of the gp39 antagonist has terminated.

12. Use of a gp39 antagonist for the preparation of a medicament for immunosuppressing function of activated helper T cells induced by a thymus-dependent (TD) antigen *in vivo*, wherein the TD antigen is administered to a subject with the gp39 antagonist.

13. The use of claim 12, wherein activated helper T cells are not deleted or are not anergized.

14. Use of a gp39 antagonist for the preparation for a composition for determining whether an antigen is a thymus-dependent (TD) antigen or a thymus-independent type II (TI-2) antigen, wherein
(a) the antigen to be tested is administered to a subject with the gp39 antagonist;
(b) humoral immune responses are measured against the antigen; and
(c) the antigen is determined to be a TD antigen by an absence of humoral immune responses or the antigen is determined to be a TI-2 antigen by a presence of humoral immune responses.

15. The composition or use of any one of claims 1 to 14, wherein the TD antigen is a protein.

16. The composition or use of any one of claims 1 to 15 wherein the TD antigen is an antibody.

17. The composition or use of any one of claims 1 to 16, wherein the TD antigen is on a cell surface.

18. The composition or use of any one of claims 1 to 7, wherein the TD antigen is a therapeutic agent.

19. The composition or use of any one of claims 1 to 18 wherein the antagonist is an anti-gp39 antibody.

20. The composition or use of claim 19, wherein the anti-gp39 antibody is a monoclonal antibody.

21. The composition or use of claim 20, wherein the anti-gp39 antibody is monoclonal antibody 24-31 (ATCC No. HB11712) or 89-76 (ATCC No. HB11713).

22. The composition or use of claim 20, wherein the anti-gp39 antibody is a chimeric monoclonal antibody.

23. The composition or use of claim 19 or 21, wherein the anti-gp39 antibody is a humanized monoclonal antibody.

24. The composition or use of any one of claims 5 to 22, wherein the gp39 antagonist is a soluble form of a gp39 ligand.

25. The composition or use of claim 24, wherein the gp39 ligand is CD40.

26. The composition or use of claim 25, wherein the CD40 is a fusion protein.

27. Use of a gp39 antagonist for the preparation of a medicament for providing for the suppression of a humoral response to an administered thymus-dependent (TD) antigen which TD antigen comprises a therapeutic agent to which a subject is exposed.

28. Use of a gp39 antagonist for the preparation of a medicament for providing for the suppression of an antigen specific IgE response to an administered thymus-dependent (TD) antigen, which TD antigen comprises a therapeutic agent to which a subject is exposed.

29. The use of claim 27, wherein the therapeutic agent is a therapeutic antibody or, a drug.

30. Use of an anti-gp39 antibody for the preparation of a medicament for preventing clearance of an administered TD antigen, wherein said administered TD antigen is a therapeutic agent.

31. The use of Claim 30, wherein said administered TD antigen is a therapeutic antibody.

## Patentansprüche

1. Pharmazeutische Zusammensetzung, umfassend ein Thymus-abhängiges (TD)-Antigen und einen gp39-Antagonisten.

2. Pharmazeutische Zusammensetzung nach Anspruch 1, in welcher das TD-Antigen ein Allergen ist.

3. Pharmazeutische Zusammensetzung nach Anspruch 2, weiter umfassend einen IL-4-Inhibitior.

4. Verwendung eines gp39-Antagonisten für die Herstellung eines Medikaments zur Inhibierung einer humoralen Immunantwort auf ein Thymus-abhängiges (TD)-Antigen in *vivo*, wobei das TD-Antigen einem Patienten mit dem Antagonisten verabreicht wird.

5. Verwendung nach Anspruch 4, wobei die humorale Immunantwort eine primäre humorale Immunantwort oder eine sekundäre humorale Immunantwort ist.

6. Verwendung nach Anspruch 4 oder 5, wobei humorale Immunantworten auf TI-2-Antigene nicht inhibiert werden.

7. Verwendung eines gp39-Antagonisten für die Herstellung eines Medikaments zur Bereitstellung von Unterdrückung einer Antigen-spezifischen IgE-Antwort auf ein verabreichtes Thymus-abhängiges (TD)-Antigen *in vivo*, wobei der gp39-Antagonist einem dem TD-Antigen ausgesetzten Patienten verabreicht wird und die Unterdrückung der Antikörper-Produktion gegen das verabreichte Antigen nach Beendigung der Verabreichung des gp39-Antagonisten aufrechterhalten wird.

8. Verwendung eines gp39-Antagonisten für die Herstellung eines Medikaments zur Bereitstellung der Unterdrückung einer humoralen Antwort auf ein verabreichtes Allergen, welchem ein Patient ausgesetzt ist, wobei die Unterdrückung der Antikörper-Produktion gegen das verabreichte Antigen nach Beendigung der Verabreichung des gp39-Antagonisten aufrechterhalten wird.

9. Verwendung nach Anspruch 8, wobei ein IL-4-Inhibitor dem Patienten koverabreicht wird.

10. Zusammensetzung nach Anspruch 3 oder Verwendung nach Anspruch 9, in welcher der IL-4-Inhibitor ein IL-4-Antikörper ist.

11. Verwendung eines gp39-Antagonisten für die Herstellung eines Medikaments zur Unterdrückung einer humoralen Immunantwort auf ein verabreichtes Thymus-abhängiges (TD)-Antigen *in vivo*, wobei das TD-Antigen einem Patienten mit dem gp39-Antagonisten verabreicht wird und die Unterdrückung der Antikörper-Produktion gegen das verabreichte Antigen nach Beendigung der Verabreichung des gp39-Antagonisten aufrechterhalten wird.

12. Verwendung eines gp39-Antagonisten für die Herstellung eines Medikaments für die Immunosuppression der Funktion von aktivierten T-Helferzellen, die durch ein Thymus-abhängiges (TD)-Antigen induziert wird, *in vivo*, wobei das TD-Antigen einem Patienten mit dem gp39-Antagonisten verabreicht wird.

13. Verwendung nach Anspruch 12, wobei aktivierte T-Helferzellen nicht zerstört oder nicht anergetisiert werden.

14. Verwendung eines gp39-Antagonisten für die Herstellung einer Zusammensetzung zur Bestimmung, ob ein Antigen ein Thymus-abhängiges (TD)-Antigen oder ein Thymus-unabhängiges Typ II (TI-2)-Antigen ist, wobei
a) das zu testende Antigen einem Patienten mit dem gp39-Antagonisten verabreicht wird,.
b) humorale Immunantworten gegen das Antigen gemessen werden; und
c) das Antigen angesichts einer Abwesenheit von humoralen Immunantworten als ein TD-Antigen bestimmt wird oder das Antigen angesichts einer Anwesenheit von humoralen Immunantworten als ein TI-2-Antigen bestimmt wird.

15. Zusammensetzung oder Verwendung nach irgendeinem der Ansprüche 1 bis 14, worin das TD-Antigen ein Protein ist.

16. Zusammensetzung oder Verwendung nach irgendeinem der Ansprüche 1 bis 15, worin das TD-Antigen ein Antikörper ist.

17. Zusammensetzung oder Verwendung nach irgendeinem der Ansprüche 1 bis 16, worin das TD-Antigen auf einer Zelloberfläche vorliegt.

18. Zusammensetzung oder Verwendung nach irgendeinem der Ansprüche 1 bis 17, worin das TD-Antigen ein therapeutisches Mittel ist.

19. Zusammensetzung oder Verwendung nach irgendeinem der Ansprüche 1 bis 18, worin der Antagonist ein anti-gp39-Antikörper ist.

20. Zusammensetzung oder Verwendung nach Anspruch 19, worin der anti-gp39-Antikörper ein monoklonaler Antikörper ist.

21. Zusammensetzung oder Verwendung nach Anspruch 20, worin der anti-gp39-Antikörper monoklonaler Antikörper 24-31 (ATCC-Nr. HB11712) oder 89-76 (ATCC-Nr. HB11713) ist.

22. Zusammensetzung oder Verwendung nach Anspruch 20, worin der anti-gp39-Antikörper ein chimerer monoklonaler Antikörper ist.

23. Zusammensetzung oder Verwendung nach Anspruch 19 oder 21, worin der anti-gp39-Antikörper ein humanisierter monoklonaler Antikörper ist.

24. Zusammensetzung oder Verwendung nach irgendeinem der Ansprüche 5 bis 22, worin der gp39-Antagonist eine lösliche Form eines gp39-Liganden ist.

25. Zusammensetzung oder Verwendung nach Anspruch 24, worin der gp39-Ligand CD40 ist.

26. Zusammensetzung oder Verwendung nach Anspruch 25, worin das CD40 ein Fusionsprotein ist.

27. Verwendung eines gp39-Antagonisten für die Herstellung eines Medikaments zur Bereitstellung der Unterdrückung einer humoralen Antwort auf ein verabreichtes Thymus-abhängiges (TD)-Antigen, wobei das TD-Antigen ein therapeutisches Mittel umfaßt, dem ein Patient ausgesetzt ist.

28. Verwendung eines gp39-Antagonisten für die Herstellung eines Medikaments zur Bereitstellung der Unterdrückung einer Antigen-spezifischen IgE-Antwort auf ein verabreichtes Thymus-abhängiges (TD)-Antigen, wobei das TD-Antigen ein therapeutisches Mittel umfaßt, dem ein Patient ausgesetzt ist.

29. Verwendung nach Anspruch 27, wobei das therapeutische Mittel ein therapeutischer Antikörper oder ein Arzneistoff ist.

30. Verwendung eines anti-gp39-Antikörpers für die Herstellung eines Medikaments zur Verhinderung von Clearance eines verabreichten TD-Antigens, wobei das verabreichte TD-Antigen ein therapeutisches Mittel ist.

31. Verwendung nach Anspruch 30, wobei das verabreichte TD-Antigen ein therapeutischer Antikörper ist.

## Revendications

1. Composition pharmaceutique comprenant un antigène dépendant du thymus (TD) et un antagoniste de gp39.

2. Composition pharmaceutique suivant la revendication 1, dans laquelle l'antigène TD est un allergène.

3. Composition pharmaceutique suivant la revendication 2, comprenant en outre un inhibiteur de IL-4.

4. Utilisation d'un antagoniste de gp39 pour la préparation des médicaments destinés à inhiber une réponse immunitaire humorale à un antigène dépendant du thymus (TD) in vivo, dans laquelle l'antigène TD est administré à un sujet avec l'antagoniste.

5. Utilisation suivant la revendication 4, dans laquelle la réponse immunitaire humorale est une réponse immunitaire humorale primaire ou une réponse immunitaire humorale secondaire.

6. Utilisation suivant la revendication 4 ou 5, dans laquelle les réponses immunitaires humorales aux antigènes TI-2 ne sont pas inhibées.

7. Utilisation d'un antagoniste de gp39 pour la préparation d'un médicament destiné à provoquer la suppression d'une réponse de IgE spécifique d'antigène à un antigène dépendant du thymus (TD) administré, in vivo, dans laquelle l'antagoniste de gp39 est administré à un sujet exposé à l'antigène TD, et la suppression de la production d'anticorps contre l'antigène administré est maintenue après la fin de l'administration de l'antagoniste de gp39.

8. Utilisation d'un antagoniste de gp39 pour la préparation d'un médicament destiné à provoquer la suppression d'une réponse humorale à un allergène administré auquel un sujet est exposé, dans laquelle la suppression de la production d'anticorps contre l'antigène administré est maintenue après la fin de l'administration de l'antagoniste de gp39.

9. Utilisation suivant la revendication 8, dans laquelle un inhibiteur de IL-4 est co-administré au sujet.

10. Composition suivant la revendication 3 ou utilisation suivant la revendication 9, dans laquelle l'inhibiteur de IL-4 est un anticorps contre la IL-4.

11. Utilisation d'un antagoniste de gp39 pour la préparation d'un médicament destiné à la suppression d'une réponse immunitaire humorale à un antigène dépendant du thymus (TD) administré in vivo, dans laquelle l'antigène TD est administré à un sujet avec l'antagoniste de gp39, et la suppression de la production d'anticorps contre l'antigène administré est maintenue après la fin de l'administration de l'antagoniste de gp39.

12. Utilisation d'un antagoniste de gp39 pour la préparation d'un médicament destiné à la fonction d'immunosuppression de lymphocytes T auxiliaires activés induite par un antigène dépendant du thymus (TD), in vivo, dans laquelle l'antigène TD est administré au sujet avec l'antagoniste de gp39.

13. Utilisation suivant la revendication 12, dans laquelle les lymphocytes T auxiliaires activés ne sont pas supprimés ou ne sont pas anergisés.

14. Utilisation d'un antagoniste de gp39 pour la préparation d'une composition permettant de déterminer si un antigène est un antigène dépendant du thymus (TD) ou un antigène de type II indépendant du thymus (TI-2), dans laquelle
(a) l'antigène à tester est administré à un sujet avec l'antagoniste de gp39 ;
(b) les réponses immunitaires humorales sont mesurées contre l'antigène ; et
(c) l'antigène est déterminé en tant qu'antigène TD par une absence de réponses immunitaires humorales ou bien l'antigène est déterminé en tant qu'antigène TI-2 par la présence de réponses immunitaires humorales.

15. Composition ou utilisation suivant l'une quelconque des revendications 1 à 14, dans laquelle l'antigène TD est une protéine.

16. Composition ou utilisation suivant l'une quelconque des revendications 1 à 15, dans laquelle l'antigène TD est un anticorps.

17. Composition ou utilisation suivant l'une quelconque des revendications 1 à 16, dans laquelle l'antigène TD est présent sur la surface d'une cellule.

18. Composition ou utilisation suivant l'une quelconque des revendications 1 à 17, dans laquelle l'antigène TD est un agent thérapeutique.

19. Composition ou utilisation suivant l'une quelconque des revendications 1 à 18, dans laquelle l'antagoniste est un anticorps anti-gp39.

20. Composition ou utilisation suivant la revendication 19, dans laquelle l'anticorps anti-gp39 est un anticorps monoclonal.

21. Composition ou utilisation suivant la revendication 20, dans laquelle l'anticorps anti-gp39 est l'anticorps monoclonal 24-31 (ATCC N° HB1172) ou 89-76 (ATCC N° HB11713).

22. Composition ou utilisation suivant la revendication 20, dans laquelle l'anticorps anti-gp39 est un anticorps monoclonal chimère.

23. Composition ou utilisation suivant la revendication 19 ou 21, dans laquelle l'anticorps anti-gp39 est un anticorps monoclonal humanisé.

24. Composition ou utilisation suivant l'une quelconque des revendications 5 à 22, dans laquelle l'antagoniste gp39 est une forme soluble d'un ligand gp39.

25. Composition ou utilisation suivant la revendication 24, dans laquelle le ligand gp39 est un ligand CD40.

26. Composition ou utilisation suivant la revendication 25, dans laquelle le ligand CD40 est une protéine de fusion.

27. Utilisation d'un antagoniste de gp39 pour la préparation d'un médicament destiné à provoquer la suppression d'une réponse humorale à un antigène dépendant du thymus (TD) administré, antigène TD qui comprend un agent thérapeutique auquel un sujet est exposé.

28. Utilisation d'un antagoniste de gp39 pour la préparation d'un médicament destiné à provoquer la suppression d'une réponse de IgE spécifique d'antigène à un antigène dépendant du thymus (TD) administré, antigène TD qui comprend un agent thérapeutique auquel un sujet est exposé.

29. Utilisation suivant la revendication 27, dans laquelle l'agent thérapeutique est un anticorps thérapeutique ou un médicament.

30. Utilisation d'un anticorps anti-gp39 pour la préparation d'un médicament destiné à empêcher la clairance d'un antigène TD administré, dans laquelle ledit antigène TD administré est un agent thérapeutique.

31. Utilisation suivant la revendication 30, dans laquelle l'antigène TD administré est un anticorps thérapeutique.
